# EUROPÄISCHE PATENTSCHRIFT

(11) **EP 2 394 617 B1**
(45) Veröffentlichungstag und Bekanntmachung des Hinweises auf die Patenterteilung: **11.12.2013**
(21) Anmeldenummer: 10165551.2
(22) Anmeldetag: 10.06.2010
(51) Int. Cl.: A61F 13/00, A61K 8/02, A61L 15/22, A61L 15/28, A61L 27/20

(54) **Schichtförmige perforierte Biomatrices**
Layer-like perforated biomatrices
Biomatrices perforées en forme de couches

(43) Veröffentlichungstag der Anmeldung: 14.12.2011
(73) Patentinhaber: MedSkin Solutions Dr. Suwelack AG, 48727 Billerbeck (DE)
(72) Erfinder: Wieland, Martin, 48653 Coesfeld (DE); Haas, Hermann, 46348 Raesfeld (DE)
(74) Vertreter: Gille Hrabal

(56) Entgegenhaltungen:
- WO-A2-2005/060550
- GB-A- 1 102 118
- US-A- 3 143 208
- US-A- 4 789 401
- US-A1- 2002 103 542
- US-A1- 2003 190 339

## Beschreibung

Die vorliegende Erfindung betrifft schichtförmige Biomatrices, aus einem Trägermaterial, umfassend mindestens ein Polymer aus der Gruppe der strukturbildenden hydrophilen natürlichen Polymere und/oder der bioabsorbierbaren synthetischen und/oder modifizierten natürlichen Polymere die aus regelmäßig geometrisch geformten, gleichmäßig angeordneten Teilstücken, die durch kontinuierliche über die Fläche der schichtförmigen Biomatrix verlaufende, Perforationen abtrennbar miteinander verbunden sind, gebildet werden, sowie deren Verwendung als kosmetisches oder pharmazeutische Mittel, wie insbesondere als Mittel zur Wundbehandlung. Weiterhin betrifft die vorliegende Erfindung ein Verfahren zur Herstellung derartiger schichtförmiger Biomatrices, sowie deren Kombination in Kit-of-parts Anordnungen.

Es sind diverse Mittel zur kosmetischen und therapeutischen Behandlung des menschlichen Körpers in den unterschiedlichsten Darreichung- und Applikationsformen bekannt. Eine wichtige Rolle spielen hierbei Masken, Sheets, Matrices, Auflagen, Pads, Lagen oder ähnliche flächige Formen, da sich solche Ausführungsformen besonders zur äußerlichen und flächigen Behandlung und Pflege der Haut sowie auch zur Versorgung von flächigen Hautverletzungen oder Wunden eignen. Je nach gewünschtem Behandlungsziel oder gewähltem Einsatzgebiet der Auflagen sind besondere, mitunter sehr spezifische Material- und Funktionsanforderungen an solche Zusammensetzungen, insbesondere an ihre chemische Zusammensetzung sowie ihre physikalische oder biologisch-chemische Wirk- und Funktionsweise, zu stellen. Speziell bei der äußerlichen dermalen Behandlung sind die komplexen biochemischen Wechselwirkungen und Funktionsweisen mit dem Organ "Haut" zu beachten.

Dabei sind nahezu gleichermaßen die Pflege und der Schutz der Haut zum Beispiel durch eine kosmetische Behandlung sowie die Wiederherstellung, Heilung oder Linderung von Funktionsstörungen oder Verletzungen der Haut durch eine therapeutische Behandlung von Bedeutung.

Die Pflege und der vorbeugende Schutz durch eine kosmetische Behandlung kann insbesondere durch die Auf- und Einbringung von Wirk-, Nähr- und/oder Pflegestoffen erzielt werden, aber auch durch Unterstützung oder Verbesserung der physikalischen und mechanischen Schutz- und/oder Barriereeigenschaften, wie Elastizität, Glätte, Rauhigkeit, Trockenheit oder biochemisches Gleichgewicht der Haut. Hier stellt insbesondere die Unterstützung, der Schutz, die Regulierung sowie Verbesserung des Feuchtigkeits- und Fettgehaltes, insbesondere des sogenannten "Natural Moisturizing Factors" (NMF) sowie der Barrierefunktion der Haut ein wichtiges Behandlungselement dar.

Bei einer Verletzung oder Beeinträchtigung der Haut oder einer ihrer zentralen Funktionen ist eine Behandlung von besonderer Bedeutung, die eine lindernde, heilende oder wiederherstetlende Wirkung mit sich bringt. Eine solche therapeutische Behandlung kann ebenfalls durch Zuführung bestimmter positiv wirkender Aktiv-, Heil- oder Wirkstoffe oder durch geeignete unterstützende physikalische oder biochemische Methoden, die die Selbstheilung unterstützen und günstig beeinflussen, erfolgen. Die Art und der Umfang einer solchen therapeutischen Behandlung sind dabei insbesondere von der Art der Verletzung oder Funktionsstörung abhängig und speziell auf die betroffenen Hautschichten abzustimmen.

Für beide Bereiche, den pflegenden kosmetischen sowie den therapeutischen Bereich der Hautbehandlung, ist die Verwendung von festen, trockenen oder vorbefeuchteten, absorbierenden bzw. hydratisierbaren Zubereitungsformen, insbesondere in Form von flächigen Masken, Sheets, Auflagen oder Pads prinzipiell besonders geeignet und auch bereits weit verbreitet. Dabei sind besonders solche Zubereitungen von Interesse, die neben der Wirkstoff-Applikation auch an sich bereits eine die Haut hydratisierende sowie gegebenenfalls kühlende Wirkung bzw. einen positiven Einfluss auf die natürlichen Wundheilungsprozesse aufweisen. Dies ist gleichermaßen relevant für die kosmetische als auch therapeutische Hautbehandlung.

Neben diesen biochemischen Gesichtspunkten sind jedoch auch die geometrischen und mechanischen Ausgestaltungen der Auflagen von großer Wichtigkeit und müssen an die jeweiligen zu behandelnden Körperregionen angepasst werden, um eine effiziente Behandlung der Haut zu ermöglichen.

Die vorliegende Erfindung betrifft nun das spezielle technische Gebiet der schichtförmigen kosmetischen und therapeutischen Auflagen bzw. Implantate und insbesondere spezifische geometrisch-mechanische Ausgestaltungen solcher schichtförmiger Auflagen.

Unter einer Auflage im Sinne der vorliegenden Erfindung versteht man dabei eine schichtförmigen Matrix, die in Form eines Sheets, Vlieses, Pads, Layers oder in der Art einer Maske oder Kompresse u.ä. ausgestaltet ist und die auf mindestens einen Teilbereich des menschlichen oder tierischen Körpers aufgebracht werden kann. Darüber hinaus wird unter einer Auflage auch ein Implantat zur Einbringung in den menschlichen oder tierischen Körper bzw. in eine Wunde verstanden, wobei derartige Implantate entweder im Körper verbleiben und ggf. physiologisch umgesetzt oder abgebaut werden oder im Rahmen der Behandlung wieder entfernt werden können. Unter einer Auflage versteht man im Rahmen der vorliegenden Erfindung darüber hinaus im Allgemein auch ein spezielles Pflege- oder Behandlungsmittel, welches mit warmen oder kalten Flüssigkeiten, die ggf. weitere Wirkstoffe enthalten, befeuchtet und auf die Haut oder die Wunde aufgebracht werden kann. Diese soll die allgemeine Hautpflege und Wundbehandlung unterstützen und ergänzen.

Schichtförmige, durchgängig, homogen flächig ausgestaltete Biomatrices sind beispielsweise in der kosmetischen Anwendung bekannt als Hautbehandlungsmittel in Form von kosmetischen Auflagen oder Gesichtsmasken.

Auch im Bereich der Wundversorgung sind diverse flächige Ausgestaltungen von Wundbehandlungsmitteln auf Basis von Biomatrices bekannt. Dabei zeichnen sich sowohl in der kosmetischen Anwendung als auch in der Verwendung als Wundauflage flächig oder schichtförmig ausgestaltete Biomatrix-Materialien insbesondere durch eine gute und gleichmäßige großflächige Applizierbarkeit und eine in der Regel gute Modellierbarkeit und Positionierbarkeit auf der zu behandelnden Hautfläche aus.

Insbesondere bei der Behandlung von Wunden ist daneben jedoch auch eine extrem hohe Flexibilität für eine räumliche bzw. dreidimensionale Modellierbarkeit des schichtförmigen Materials wünschenswert, um insbesondere bei der Behandlung von Wunden mit tiefen Hautdefekten oder großen Wundhöhlen eine möglichst vollständige und homogene Ausfüllung bzw. Auskleidung oder Tamponade solcher tieferen Hautdefekte oder Wundhöhlen zu erreichen.

Üblicherweise finden zur Auskleidung tiefer Wunden bisher häufig pulverförmige oder gelförmige Wundbehandlungssysteme Anwendung. Aus der Gruppe der Pulver sind dabei insbesondere Mittel wie Avitene^{®} Flour als Hämostyptikum der Firma Davol Inc. oder Orahesive^{®} von ConvaTec als Mittel für die Wundbehandlung bekannt. Als Wundbehandlungsmittel in Gelform werden üblicherweise solche auf Basis von Flydrogelbildendon Polysacchariden eingesetzt. Bekannte Beispiele sind hier Askina Gel® von B. Braun, Hydrosorb® Gel von Hartmann, Varihesive Hydrogel® von ConvaTech und zahlreiche andere.

Pulver und Gele als Mittel in der Behandlung von Wunden oder als Hämostyptika weisen den Nachteil auf, daß sie sich zwar einerseits gut in tiefe Wunden oder Hautdefekte einstreuen oder einbringen lassen, dort allerdings nach Kontakt mit der Wundflüssigkeit oder der feuchten Oberfläche des behandelten Körperareals nur schwer bis gar nicht in ihrer Positionierung korrigiert werden können, da sie meist mit dem Wundgrund verkleben oder daran adhärieren. Eine gleichmäßige, flächige Aufbringung ist insbesondere bei der Verwendung von pulverförmigen Wundbehandlungsmitteln nur schwer möglich und hängt in der Regel stark vom Geschick und der Erfahrung des Anwenders ab.

Ähnliche Probleme zeigen sich bei der Verwendung von gelförmigen Wundbehandlungsmitteln, die sich zwar gut an den Wundoberflächen anmodellieren lassen, jedoch nur bedingt homogen auf die Wundflächen aufgebracht werden können. Darüber hinaus besteht bei gelförmigen Zubereitungen, bedingt durch den nicht unerheblichen Wassergehalt solcher Zubereitungen, immer das Problem der Stabilisierung und Konservierung gegen mikrobiellen Verderb. Dies bedingt einerseits besondere zusätzliche Aufwendungen bei der Herstellung solcher Produkte und birgt außerdem insbesondere bei Verwendung chemischer Konservierungsstoffe das Risiko unerwünschter Nebenwirkungen oder Unverträglichkeitsreaktionen bei der Anwendung.

Bei der Verwendung bekannter und gebräuchlicher schichtförmiger Biomatrices in Form flächiger Ausgestaltungen z.B. in Form von Sheets, Vliesen, Kompressen, Pads u.ä. handelt es sich in der Regel um durchgängige, einheitliche schichtförmige Vliese oder Auflagen, die in den verschiedensten geometrischen Formen wie z. B. Rechtecke, Kreise u.a. ausgebildet oder zugeschnitten sein können. Beispielsweise sind flächige oder schichtförmige durchgängige Wundauflagen auf Basis von Collagen aus dem Bereich der Behandlung von chronischen Wunden oder der Hämostyptika bekannt unter der Bezeichnung Matriderm^{®}, Matristypt^{®} oder Puracol^{®} von der Firma Dr. Suwelack Skin & Health Care AG oder als Suprasorb^{®} von der Firma Lohmann & Rauscher, als Promogran^{®} von der Firma Johnson & Johnson bzw. Systagenics oder als Avitene^{®} Sheets von Davol Inc. Des weiteren sind bekannte Beispiele flächiger, schichtförmiger Wundauflagen auf Basis von Polysacchariden u.a. Algisite M^{®} von Smith & Nephew, Askina Sorb^{®} von B. Braun sowie zahlreiche weitere. Auch flächige, sheetförmige Wundauflagen auf Basis von anderen Polysacchariden wie z. B. Chitoskin^{®} von Sangui BioTech GmbH oder Mischungen von z. B. Collagen und Alginat wie z. B. Fibracol^{®} von Johnson & Johnson sind bekannt und werden als gängige Wundbehandlungsmittel sowohl bei chronischen Wunden als auch als Hämostyptika oder blutstillende Mittel eingesetzt.

Solche einheitlich bzw. durchgängig homogen-flächig ausgestalteten Wundauflagen auf Basis von Biomatrix-Materialien weisen in der Regel nur eine bedingte Flexibilität auf und können nur in begrenztem Maße homogen und vollständig-ausfüllend in Wundhöhlen oder tiefe Wunden eingepasst werden. Somit können tiefe und unregelmäßig ausgebildete Wundhöhlen meist nur unzureichend und unvollständig mit solchen einheitlich flächig gestalteten Biomatrix-Wundbehandlungsmaterialien ausgekleidet werden und selbst bei Verwendung eines sehr flexiblen Vlieses kann in der Regel keine vollständige Tamponade der Wunde erhalten werden.

Des weiteren ist es häufig notwendig, die vorgefertigten flächig ausgestalteten Wundbehandlungsmittel z.B. solche Sheet-, Layer- oder Schicht-förmigen Wundauflagen an unregelmäßig geformte Wundränder anzupassen. Dies geschieht bei den bekannten einheitlich, homogen-flächig ausgebildeten Materialien in der Regel durch Zuschneiden vor der Applikation. Dies stellt jedoch einerseits einen zusätzlichen Arbeitsaufwand dar und bringt außerdem ein erhöhtes Infektions- und Verletzungsrisiko, bedingt durch die Handhabung und Inkontaktbringung der Wundauflagen mit Schneidwerkzeugen wie Scheren, Messern etc., mit sich.

Insgesamt ist mit durchgängig homogen-flächig ausgestalteten schichtförmigen Wundbehandlungsmaterialien, wie sie bisher bekannt und gebräuchlich sind, eine gleichmäßige und homogene Aufbringung auf flächigen sowie eine vollständige und homogene räumliche Auskleidung tiefer Wundhöhlen nur unzureichend möglich und solche Materialien sind darüber hinaus mit dem Nachteil einer weiteren Formanpassung durch Anwendung von mechanischen Schneidwerkzeugen verbunden.

Überraschend wurde gefunden, daß bekannte flächig oder schichtförmig ausgestaltete Biomatrices wie z. B. solche auf Basis von Collagen, aber auch solche auf Basis natürlicher pflanzlicher Hydrokolloide in ihrem Applikation-, Modulier- und Absorptionsverhalten deutlich verbessert werden können, indem sie mit kontinuierlichen, durchgehenden Perforation versehen werden, wobei diese Perforationen so angebracht werden, dass dadurch eine schichtförmige Biomatrix gebildet wird, die insgesamt aus miteinander verbundenen, regelmäßig geformten, gleichmäßig angeordneten Teilstücken gebildet wird.

Ein weiterer Vorteil, der sich aus dieser speziellen Perforationstechnik ergibt, liegt darin, daß derartige aus miteinander verbundenen regelmäßig geformten, gleichmäßig angeordneten Teilstücken gebildete schichtförmige Biomatrices leicht und schnell durch einfaches Abreißen bzw. Abtrennen entlang der Perforationen in nahezu jede gewünschte und benötigte Größe gebracht werden können, ohne dass der zusätzliche und aus genannten Gründen als nachteilig einzustufende Einsatz von Schneidwerkzeugen notwendig ist.

Aus dem Stand der Technik sind flächige oder schichtförmige Biomatrices, die Perforationen aufweisen, prinzipiell bekannt.

Dabei werden insbesondere im Bereich der Wundbehandlungsmittel Verbandmaterialien, Folien oder Biomatrices häufig mit einer durch die gesamte Dicke des Materials durchgehenden punkt- oder lochförmigen Perforation versehen, wodurch das Material eine Art Sieb- bzw. Loch- oder Porenstruktur erhält. Eine derartige Durchlöcherung der Verbandsmaterialien dient dazu, den Gas- und/oder Flüssigkeitsdurchtritt durch die Materialien zu erhöhen bzw. zu ermöglichen. Derartige punktförmig durchperforierte Matrices sind beispielsweise beschrieben in der DE 1642012, US 2002/0103542, US 2003/0190339, US 5060678.

Beispielsweise offenbart die US 2002/0103542 Collagen-Layer und Multilayer-Materialien aus aufgereinigtem intestinalem azellulärem telopeptid Typ I Collagen (ICL) und dessen Verwendung zur Wundbehandlung oder als Implantat. Die Collagen-Layer können außerdem perforiert, "gefenstert", durchlöchert oder in Form eines Mesh vorliegen. Derartige Perforationen sind dabei insbesondere dann anzubringen, wenn das an sich unporöse Material zwecks Flüssigkeitsdurchtritt durchlässig gemacht werden soll. Weiterhin ist beschrieben, dass die Collagen-Layer in Streifen von bis zu 15 cm Länge bzw. in Stücke von ca. 15 x 15 cm geschnitten werden.

Die US 2003/0190339 betrifft schichtförmige Matrixmaterialien aus dem Bereich der Wundbehandlungsmittel wie Verbandmaterialien, Folien oder Biomatrices die mit einer durch die gesamte Dicke des Materials durchgehenden punkt- oder lochförmigen Perforation versehen sind.

Die DE 1642012 bzw. GB 1102118 offenbart Collagenschwämme als Verbandmaterialien, welche mit Perforationen in Form einer Durchlöcherung versehen sein können.

Die US 4,789,401 betrifft lösliche Collagenschwämme, wobei eine Perforation dieser Collagenschwämme nicht erwähnt wird.

Die WO 2005/060550 betrifft eine Wund-Debridement-Zusammensetzung, welche eine Hydrogelauflage (Hydrogelpatch) enthält. Eine Perforierung dieses "Hydrogelpatches" wird nicht erwähnt.

Außerdem ist es Insbesondere im Bereich der Wundverbandsmaterialien bekannt und üblich, eine Art Perforation in Form von schlitzartig angebrachten Einschnitten in einem Matrixmaterial auszubilden, wodurch eine Art Netzstruktur der Matrices entsteht. Ein derart geschlitztes Material wird üblicherweise auch als gemeshtes Material bezeichnet. Dabei ist das sogenannte "mesh"-Verfahren insbesondere bekannt aus der Bearbeitung von Spalthaut, die durch das Anbringen der regelmäßig angeordneten schlitzartigen Einschnitte auf ein Vierfaches ihrer Flächengröße aufgezogen werden kann, vergleichbar mit einem herkömmlichen Einkaufsnetz. Dadurch wird es möglich, mit vergleichsweise kleinen Spalthautstücken eine um ein mehrfaches größere Transplantationsfläche zu bedecken. Eine derartige mesh- oder netzartige Schlitz-Perforation von Biomatrices ist beispielsweise bekannt aus der US 4520821 oder aus der US 6183496 und US 6281309 oder auch aus der WO 03/035125.

Dabei bilden jedoch weder punkt- oder schlitz-förmig perforierte bzw. gemeshete Matrix-Materialien regelmäßig geformte, gleichmäßig angeordnete, miteinander verbundene Teilstücke aus. Die Bildung derartiger miteinander verbundener Teilstücke ist jedoch maßgeblich für die oben beschriebene Verbesserung der Flexibilität, insbesondere für eine räumliche Modelllerbarkeit, sowie zur einfachen Größen- und Formanpassung der schichtförmigen Matrices.

Biomatrices mit durchgänglgen Perforationen, wodurch die Ausbildung von Teilstücken erfolgt, sind im Prinzip auch durch die EP 1272158 B1 offenbart. Gegenstand dieser Patentschrift sind Gesichtsmasken aus einem flexiblen, zur Aufnahme von Flüssigkeiten geeigneten bzw. aufnehmenden Träger, der aus wenigstens einer bahnförmigen Komponente besteht rund der dadurch gekennzeichnet ist, daß wenigstens eine der Komponenten aus wenigstens zwei Teilkomponenten besteht, die lösbar miteinander verbunden sind. Bei derartigen Masken handelt es sich beispielsweise um solche auf der Basis von Collagen, sowie um solche, die zur Verwendung als kosmetische Hautbehandlungsmittel vorgesehen sind. Als erfindungsgemäßer Vorteil wird die einfachere Applikation ohne aufwändiges Zuschneiden auf die zu behandelnde Körperpartie, insbesondere das Gesicht hervorgehoben. Dabei werden durch die Perforationen Teilkomponenten gebildet, die die Form der unterschiedlichen Gesichtsareale aufweisen. Die dort offenbarten Teilkomponenten sind jedoch nicht regelmäßig geformt und gleichmäßig angeordnet. Außerdem sind die durch die dort offenbarten Perforationen gebildeten Teilkomponenten vergleichsweise groß, da sie zur Abdeckung größerer Behandlungsareale vorgesehen sind. Dadurch kann jedoch keine Erhöhung der Flexibilität und damit der Verbesserung der räumlichen Modellierbarkeit erreicht werden, wie sie durch die Bildung regelmäßig geformter, gleichmäßig angeordneter Teilstücke von vergleichsweise geringer Größe, gemäß der vorliegenden Erfindung, möglich ist. Auch ergeben sich aus der genannten Patentschrift keine Hinweise auf eine Verwendung der beschriebenen Masken als therapeutisches Mittel insbesondere zur Wundbehandlung oder als Hämostyptikum. Die mit dieser besonderen Perforationstechnik der vorliegenden Erfindung erzielte Verbesserung der Flexibilität und räumlichen Modellierbarkeit von schichtförmigen Biomatrices kann der EP 1272158 B1 damit nicht entnommen werden.

Aus der US 3143208 sind adhäsive Pflastermaterialien bekannt, die kontinuierliche, durchgehende Perforationen unter Ausbildung regelmäßig geformter, gleichmäßig angeordneter, miteinander verbundener Teilstücke aufweisen. Durch derart angebrachte Perforationen wird die Abtrennung von Teilstücken unterschiedlicher, individuell gewünschter Größe ermöglicht. Bei den dort beschriebenen perforierten Materialien handelt es sich jedoch nicht um Biomatrices und insbesondere nicht um solche Materialien, die als Behandlungsmittel auf die Haut oder auf bzw. in einer Wunde aufgebracht werden bzw. als hydrophiles Material über eine Flüssigkeitsaufnahme- oder -haltekapazität verfügen, um für die klassische Wundbehandlung geeignet zu sein. Vielmehr sind synthetische Abdeckmaterialien in Form von selbstklebenden Pflastern Gegenstand dieser Patentschrift. Die durch die Perforationen gebildeten Teilstücke weisen außerdem ebenfalls eine vergleichsweise große Ausgestaltung auf. Als kleinste Teilkomponenten werden Stücke bis minimal 2 x 0,45 inch, entsprechend ca. 6,45 cm², beschrieben. Dadurch ergeben sich auch aus dieser Patentschrift weder Hinweise auf die mit dieser Perforationstechnik erzielbare Verbesserung der Flexibilität und räumlichen Modellierbarkeit von schichtförmigen Biomatrices noch können hierin Hinweise auf die besonders geeignete Verwendung derart perforierter Biomatrix-Materialien als Haut- oder Wundbehandlungsmittel mit hoher Flüssigkeitsaufnahme- bzw. -haltekapazität gefunden werden.

Wundbehandlungsmaterialien mit einer hohen Flexibilität und räumlichen Modellierbarkeit zur Auskleidung von Wundkavitäten und tiefen Hautdefekten werden beispielsweise in den Patenten US 5928174, US 6355858 und US 6605751 beschrieben, wobei hier die verbesserte Flexibilität und räumliche Modellierbarkeit zur Ausfüllung der Wundhöhlen durch das Anbringen langer Einschnitte zur Ausbildung kammartiger Schätzungen bzw. "frei-fließender" Materialstränge erzielt wird. Dabei können solche kamm-oder strangartig eingeschnittenen Materialien in ihrer flächigen Ausgestaltung nicht ohne weiteres variiert oder angepasst werden. Auch eine gute flächige bzw. oberflächliche Aufbringung auf eine zu behandelnde Hautpartie und die dortige Remodulier- und gleichmäßige Applizierbarkeit ist durch solche geschlitzte Materialien kaum möglich.

Ein ähnlicher Effekt wird durch die Anbringung einer durchgehenden spiralförmigen Perforation zur Auftrennung des Wundbehandlungsmaterials in Form langer Streifen bzw. Einzelbänder, wie in der US 5885237 beschrieben, erzielt. Dabei kann jedoch auch eine strangartig aufgetrennte Perforation nur sehr eingeschränkt in ihrer flächigen Ausgestaltung angepasst werden, in der Regel kann maximal eine Variation der Stranglänge erfolgen, oder durch Abtrennen von Strangteilen kann eine Verkleinerung des Durchmessers der spiralförmig aufgewickelten Stränge erzielt werden. Eine Variation in der geometrischen Flächenform ist hier jedoch ebenfalls nicht ohne weiteres möglich. Aus der US 5885237 ergibt sich außerdem, dass durch die Ausbildung solcher strang- oder bandförmig ausgebildeten Matrices nur eine unzureichende, lückenhafte Wundtamponade möglich ist. Aus diesem Grund werden als Biomatrix-Materialien solche verwendet, die über eine extrem hohe Quellfähigkeit verfügen, wodurch bei Flüssigkeitsaufnahme eine deutliche Volumenvergrößerung des Matrixmaterials erfolgt, wodurch die verbliebenen Lücken ausgefüllt werden können. Bei derartig hoch-quellbaren Matrixmaterialien handelt es sich um synthetische, quellbare Hydrokolloide wie z.B. Acrylate gemäß der US 5928174, US 6355858 und US 6505751 oder um synthetische Block-Copolymere wie in der US 5885237, die ggf. mit weiteren z.B. natürlichen Polymeren gemischt werden können. Nachteilig an solchen Matrixmaterialien auf Basis synthetischer Polymere ist einerseits die fehlende biologische Abbaubarkeit, was besonders bei Implantat-Materialien eine große Rolle spielt, sowie die in der Regel geringere biologische Verträglichkeit. Weiterhin ist bei Verwendung solcher hoch-quellbaren synthetischen Matrixmaterialien der Grad der Quellung nicht bzw. nur schwer steuerbar. Dadurch kann es bei einer zu hohen Quellung in der tamponierten Wundhöhle zu einem unerwünschten Überschuss an Wundbehandlungsmaterial und dadurch ggf. zu einer Erhöhung des Drucks in der Wundhöhle aufgrund der überschießenden Volumenzunahme kommen, was sich negativ auf die Behandlung auswirkt, da dadurch einerseits die Wundheilung gestört und andererseits der Patient unnötigen Wundschmerzen ausgesetzt wird.

Die aus dem Stand der Technik bekannten schichtförmigen und ggf. perforierten oder geschlitzten Auflagen verfügen somit nicht über eine ausreichende Flexibilität für eine hohe räumliche Modellier- bzw. Modulierbarkeit zur optimalen Auskleidung von Wundkavitäten und tiefen Hautdefekten bei gleichzeitiger guter flächiger Applizierbarkeit und Modellier- bzw. Remodulierbarkeit bei gleichzeitiger einfacher und variabler flächiger Formanpassung und hoher biologischer Verträglichkeit und Degradierbarkeit, um sowohl als kosmetisches Hautpflegemittel sowie als therapeutisches Wundbehandlungsmittel und Implantat geeignet zu sein.

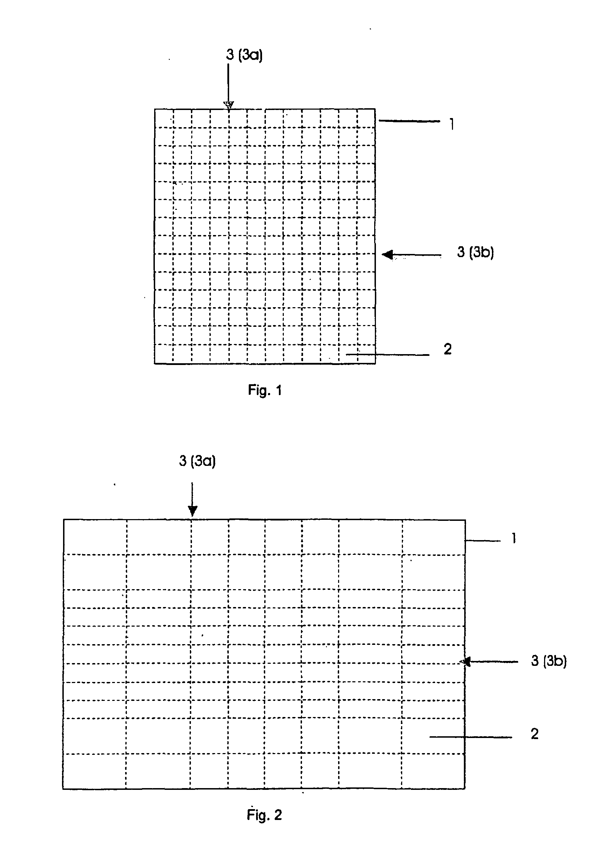

Die vorliegende Erfindung stellt sich nun die Aufgabe, die vorstehend bezeichneten Nachteile zu lösen und geht zur Lösung dieser Aufgaben von einer Auflage in Form einer schichtförmigen Biomatrix (1) aus. Die erfindungsgemäße schichtförmige Biomatrix ist in den Figuren 1 bis 12, auf welche im Folgenden Bezug genommen wird, im Detail dargestellt.

Die erfindungsgemäße schichtförmige Biomatrix (1) ist dabei dadurch gekennzeichnet, dass sie aus regelmäßig geometrisch geformten, gleichmäßig angeordneten Teilstücken (2), die durch kontinuierliche, über die Fläche der schichtförmigen Biomatrix verlaufende Perforationen (3) abtrennbar miteinander verbunden sind, gebildet wird.

Dabei bezeichnet eine schichtförmige Biomatrix im Sinne der vorliegenden Erfindung eine aus einem im wesentlichen biokompatiblen Trägermaterial gebildete Auflage, die in Form einer Schicht, eines Sheets, Vlieses, Pads, Layers oder in der Art einer Maske oder Kompresse u.ä. ausgestaltet ist und die eine im wesentlichen flächige Ausgestaltung aufweist. Dabei können solche schichtförmigen Biomatrices prinzipiell auch aus mehreren aufeinanderliegenden Schichten in der Art eines sogenannten "sandwich"layers" zusammengesetzt sein. Erfindungsgemäß bezeichnet eine schichtförmige Biomatrix insbesondere solche Auflagen im vorstehend genannten Sinne, die eine Schichtdicke (kürzeste Seitenlänge) von maximal 8 mm bzw. insgesamt eine Fläche (Fläche zwischen zwei längsten Seitenlängen) von mindestens 9 cm² aufweist.

Die durch die Perforationen (3) miteinander verbundenen regelmäßig geometrisch geformten, gleichmäßig angeordneten Teilstücke (2) der erfindungsgemäßen schichtförmigen Biomatrix sind dabei im wesentlichen dreieck-, viereck- waben-, kreis- oder ellipsenförmig ausgebildet.

Dabei umfasst im Sinne der vorliegenden Erfindung eine kreis- oder ellipsenförmige Ausgestaltung prinzipiell auch ovale geometrische Formen und eine viereckige Ausgestaltung der Teilstücke umfasst prinzipiell alle bekannten geometrischen Viereck-Formen. Insbesondere sind darunter Vierecke mit sich gegenüberliegenden parallelen und gleich langen Seiten, wie Parallelogramme, insbesondere gleichwinkelige Parallelogramme wie Rechtecke oder Quadrate, sowie Rauten oder Rhomben oder Trapeze erfasst. Aus der Gruppe der viereckig ausgebildeten Teilstücke (2) sind bevorzugt solche in Form von Rechtecken, Quadraten, Rhomben oder Parallelogrammen, wobei Rechtecke und Quadrate besonders bevorzugt sind.

Darüber hinaus können durch entsprechende Ausgestaltung der Perforationen jedoch auch Teilstücke in jeglicher anderen denkbaren geometrischen Form oder auch in Phantasieformen gebildet werden. So ist beispielsweise ebenfalls denkbar, die Teilstücke (2) in optisch bzw. ästhetisch ansprechenden Formen auszugestalten wie z.B. herz- oder sternförmig etc.

In einer bevorzugten Ausführungsform sind die Teilstücke (2) jedoch in üblichen geometrischen Formen ausgebildet, wobei eine im wesentlichen viereckige Form besonders bevorzugt ist.

Im Sinne der vorliegenden Erfindung wird unter regelmäßig geformten Teilstücken verstanden, dass mehrere der durch die Perforationen miteinander verbundenen Teilstücke (2) mit im wesentlichen gleicher geometrischer Ausgestaltung oder Form mindestens ein zusammenhängendes, durch diese gleichförmig gestalteten Teilstücke gebildetes Areal der Biomatrix (1) bilden, dass also alle Teilstücke eines solchen Areals z.B. eine rechteckige oder runde Form etc. aufweisen.

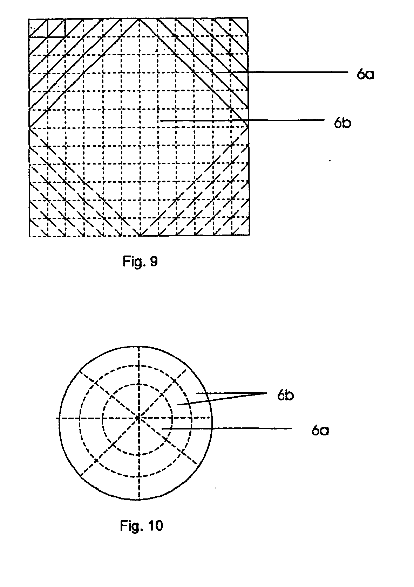

Bevorzugt weisen alle der durch die Perforationen miteinander verbundenen Teilstücke der schichtförmigen Biomatrix (1) die im wesentlichen gleiche geometrische Ausgestaltung oder Form auf, wie in den Figuren 1 bis 8 beispielhaft dargestellt, so dass in solchen Ausführungsformen quasi lediglich eines der oben beschriebenen Areale vorliegt. Es ist jedoch auch möglich, in einer Biomatrix (1) durch entsprechend angebrachte Perforationen unterschiedliche Areale (6a und 6b), die in der Regel wiederum gleichmäßig in der schichtförmigen Biomatrix (1) angeordnet sind, mit jeweils gleichförmigen regelmäßig geformten Teilstücken im oben genannten Sinne auszubilden, wodurch schichtförmige Biomatrices (1) entstehen, worin insgesamt Teilstücke unterschiedlicher geometrischer Formen innerhalb eines Sheets gebildet sein können. Eine entsprechende Ausführungsform ist beispielhaft in Figur 9 oder 10 dargestellt.

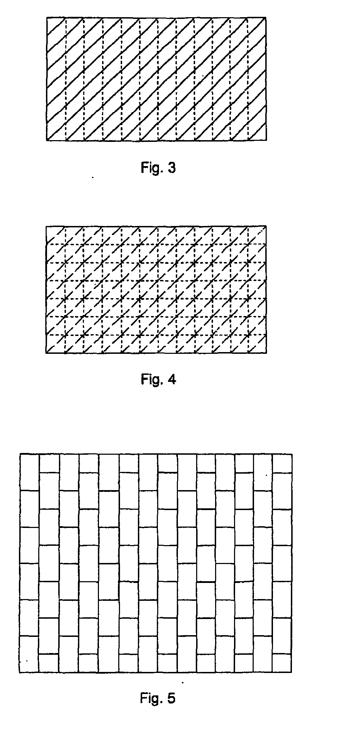

Klarstellend sei angemerkt, dass der erfindungsgemäß verwendete Begriff der "regelmäßig geformten Teilstücke" nicht im Zusammenhang mit der Größe der Teilstücke zu verstehen ist, dass also insbesondere "regelmäßig geformt" nicht eine einheitliche Größe aller die schichtförmige Biomatrix bildenden Teilstücke meint. Auch schließt "regelmäßig geformt" im vorstehend genannten Sinne nicht aus, dass einige der einheitlich geformten Teilstücke, insbesondere in den äußeren Randbereichen, durch das Zuschneiden der gesamten schichtförmigen Biomatrix teilweise beschnitten werden und somit nicht mehr vollständig in der schichtförmigen Biomatrix enthalten sein können, wie beispielsweise in Ausführungsformen nach Figuren 5 bis 8 erkennbar.

Gleichmäßig angeordnet im Sinne der vorliegenden Erfindung meint, daß die durch die Perforationen gebildeten regelmäßig geformten Teilstücke (2) in gleichmäßigen, wiederkehrenden Abständen und in einheitlicher, wiederkehrender Ausrichtung in der schichtförmigen Biomatrix (1) bzw. in dem von ihnen entsprechend gebildeten Areal der schichtförmigen Biomatrix (1) angeordnet sind.

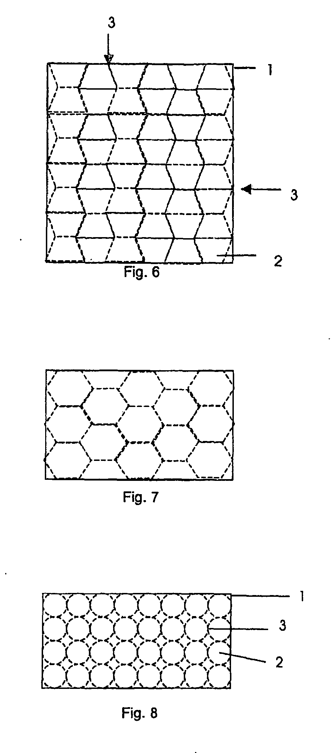

Die die schichtförmige Biomatrix (1) bildenden Teilstücke (2) sind durch kontinuierliche, durchgehende Perforationen (3) miteinander verbunden bzw. werden durch diese Perforationen aus der schichtförmigen Biomatrix gebildet. Dabei bezeichnen kontinuierliche Perforationen lückenlose, ununterbrochene, gleichmäßige Perforationen, die über die Fläche der schichtförmigen Biomatrix in einheitlicher Form verlaufen und damit in mindestens einer Richtung über die Fläche der schichtförmigen Biomatrix fortlaufende Perforationen einheitlicher Form bilden. Bevorzugt verlaufen mindestens zwei kontinuierliche, durchgehende Perforationen in im wesentlichen paralleler Anordnung zueinander über die Fläche der schichtförmigen Biomatrix. Die kontinuierlichen, durchgehenden Perforationen sind dabei bevorzugt linear ausgebildet, wodurch im wesentlichen dreieckige oder viereckige Teilstücke wie Parallelogramme, Rechtecke oder Quadrate gebildet werden, wie beispielsweise durch die Figuren 1 bis 4 oder 9 und 10 dargestellt. Weiterhin können die kontinuierlichen Perforationen auch einen im wesentlichen zick-zack-förmigen Verlauf oder auch Kombinationen aus zick-zack und linearen Verlaufsarten aufweisen, wie bei der Bildung von im wesentlichen trapez- oder wabenförmigen Teilstücken, was durch die Figuren 6 und 7 beispielhaft dargestellt ist. Insbesondere bei Ausbildung kreis- oder ellipsenförmiger Teilstücke (2) sind die kontinuierlichen Perforationen (3) wellenförmig-, kurven- oder bogenförmig ausgebildet, wie beispielsweise in Figur 8 dargestellt.

Durchgehende Perforationen bezeichnen einen gleichmäßigen, im wesentlichen gleichförmig ausgestalteten Verlauf der Perforation über die Fläche der schichtförmigen Biomatrix von einem Punkt des äußeren Randes der schichtförmigen Biomatrix zu einem diesem Punkt im wesentlichen gegenüberliegenden weiteren Punkt des äußeren Randes der schichtförmigen Biomatrix. Dabei verläuft im Sinne der vorliegenden Erfindung die kontinuierliche, durchgehende Perforation in mindestens einer Richtung der Fläche der schichtförmigen Biomatrix ununterbrochen und lückenlos von äußerem Rand zu äußerem Rand.

Bevorzugt kann eine derartige kontinuierliche, durchgehende Perforation auf im wesentlichen viereckig ausgebildeten schichtförmigen Biomatrices über die gesamte Länge, Breite bzw. in diagonaler Richtung der Fläche verlaufen. Die Länge bezeichnet dabei die längste Entfernung zweier Punkte. Bei z.B. kreisförmig gestalteten schichtförmigen Biomatrices verlaufen die kontinuierlichen, durchgehenden Perforationen bevorzugt parallel entlang des Durchmessers.

Dabei verlaufen bevorzugt mindestens zwei gleichförmige, parallel zueinander angeordnete kontinuierliche Perforationen (3a) in einer Richtung der Fläche der schichtförmigen Biomatrix und mindestens zwei weitere zueinander parallel angeordnete kontinuierliche gleichförmige Perforationen (3b), die gleich oder verschieden wie die Perforationen (3a) ausgestaltet sein können, verlaufen in mindestens einer weiteren Richtung der Fläche der schichtförmigen Biomatrix derart, dass die jeweils zueinander parallel angeordneten kontinuierlichen Perforationen (3a) die jeweils zueinander parallel angeordneten kontinuierlichen Perforationen (3b) schneiden und dadurch die regelmäßig geformten Teilstücke (2) ausbilden. Werden die durchgehenden parallelen kontinuierlichen Perforationen in lediglich zwei unterschiedlichen, sich schneidenden Richtungen der Fläche der schichtförmigen Biomatrix angebracht, so können insbesondere viereckige Teilstücke (2) gebildet werden, wie beispielsweise in den Figuren 1 bis 3 dargestellt.

In einer besonders bevorzugten Ausführungsform werden dabei die Teilstücke (2) der schichtförmigen Biomatrix (1) aus kontinuierlichen linearen, zueinander parallel angeordneten durchgehenden Perforationen (3a) und, die Perforationen (3a) schneidenden, kontinuierlichen linearen, zueinander parallel angeordneten durchgehenden Perforationen (3b) gebildet. Werden die linearen, zueinander parallel angeordneten durchgehenden Perforationen (3a) in einem rechten Winkel (90° Winkel) von den kontinuierlichen linearen, zueinander parallel angeordneten durchgehenden Perforationen (3b) geschnitten, so werden dadurch im wesentlichen rechteckige Teilstücke (2) gebildet, wie beispielhaft in den Figuren 1 und 2 dargestellt. Es ist jedoch auch möglich, die sich schneidenden Perforationen (3a) und (3b) in einem größeren oder kleineren Winkel, beispielsweise in einem Winkel von 45°, zueinander anzuordnen, wodurch Teilstücke (2) in Form von Parallelogrammen erhalten werden können, wie beispielhaft in Figur 3 dargestellt.

Um dreieckige Teilstücke (2) zu bilden, werden bevorzugt durchgehende, kontinuierliche parallele Perforationen in drei unterschiedlichen, sich jeweils schneidenden Verläufen über die Fläche der schichtförmigen Biomatrix zueinander angeordnet, wie z.B. in Figur 4 oder 9 dargestellt.

Auch ist es möglich, mindestens zwei zueinander parallel angeordnete kontinuierliche durchgehende Perforationen über die Fläche der schichtförmigen Biomatrix verlaufen zu lassen und zu diesen parallelen Perforationen rechtwinkelig angeordnet parallele, gleichmäßig angeordnete unterbrochene Perforationen derart anzubringen, dass zueinander versetzt angeordnete rechteckige Teilstücke gebildet werden, wodurch ein Muster entsteht, das mit einem Mauerverband nach Art eines Läufer- oder Binderverbandes, vergleichbar ist. Eine derartige Ausführungsform ist beispielhaft in Figur 5 dargestellt.

Erfindungsgemäß, bevorzugt sind schichtförmige Biomatrices, worin die sich schneidenden Perforationen regelmäßige, in gleichförmigen Reihen angeordnete im wesentlichen rechteckige oder dreieckige Teilstücke (2) ausbilden.

Prinzipiell ist es möglich, die Abstände zwischen den zueinander im wesentlichen parallel angeordneten kontinuierlichen Perforationen der schichtförmigen Biomatrix jeweils gleich oder verschieden auszubilden und dadurch die Größe der durch die Perforationen gebildeten Teilstücke im wesentlichen durch die Ausbildung bzw. den Abstand der kontinuierlichen Perforationen zueinander zu steuern. Dabei ist beispielsweise möglich, die Abstände zwischen den kontinuierlichen, parallel zueinander angeordneten Perforationen über die Breite bzw. Länge oder auch über die Diagonale der schichtförmigen Biomatrix so zu variieren, dass regelmäßig geformte, gleichmäßig angeordnete Teilstücke von unterschiedlicher Größe erhalten werden. Es ist jedoch auch möglich, die Abstände der jeweils zueinander parallel angeordneten kontinuierlichen Perforationen über die gesamte Fläche der schichtförmigen Biomatrix gleich auszugestalten, so dass Teilstücke (2) einheitlicher Größe entstehen. Dadurch ist es möglich, schichtförmige Biomatrices zu erhalten, worin die Größe der Teilstücke (2) jeweils gleich oder verschieden ist.

Insbesondere ist es möglich, bei bevorzugten Ausführungsformen, wie oben dargestellt, worin die Teilstücke (2) aus kontinuierlichen linearen, zueinander parallel angeordneten durchgehenden Perforationen (3a) und, die Perforationen (3a) schneidenden, kontinuierlichen linearen, zueinander parallel angeordneten durchgehenden Perforationen (3b) gebildet werden, die Abstände zwischen den parallel angeordneten Perforationen (3a) und/oder zwischen den, die Perforationen (3a) schneidenden, parallel angeordneten Perforationen (3b) jeweils gleich oder verschieden auszugestalten. Dadurch können schichtförmige Biomatrices gebildet werden, die ein über die Fläche in der Größe der Teilstücke variierendes Muster aufweisen, wie beispielhaft in Figur 2 dargestellt.

Durch eine derartige Variation der Teilstückgröße innerhalb einer schichtförmigen Biomatrix wird es möglich, deren Eigenschaften hinsichtlich Stabilität und Flexibilität durch geeignete Auswahl und Kombination der Teilstückgrößen innerhalb einer schichtförmigen Biomatrix zu variieren und in Abstimmung auf die gewünschte Anwendung optimal zu steuern.

So ist es beispielsweise möglich, die Teilstücke im äußeren Bereich der schichtförmigen Biomatrix größer auszugestalten, als die innenliegenden Teilstücke, wie z.B. in Figur 2 dargestellt. Dadurch kann eine gezielte Steuerung der Flexibilitäts- und Moduliereigenschaften der schichtförmigen Matrices erreicht werden. So bedingen größere Teilstücke eine höhere mechanische Stabilität und verbesserte Reißfestigkeit der schichtförmigen Biomatrices bei der Handhabung und Applikation, wohingegen besonders kleine Teilstücke die Flexibilität und damit verbunden die räumliche Modulierbarkeit verbessern. Durch Kombination größerer, mechanisch stabilerer Teilstücke im äußeren Bereich der Matrices und kleinerer, flexiblerer Teilstücke im innenliegenden Bereich können die Merkmale Stabilität und Flexibilität in einer schichtförmigen Biomatrix kombiniert und Materialien mit hoher Flexibilität und dennoch guter Handhabbarkeit erhalten werden.

Die regelmäßig geformten Teilstücke der erfindungsgemäßen Biomatrices weisen eine Größe von maximal 5 cm², bevorzugt von maximal 4 cm², besonders bevorzugt maximal 3 cm² auf. Bevorzugt weisen die regelmäßig geformten Teilstücke eine Größe von ca. 0,75 cm², bevorzugt von ca. 0,5 cm², bevorzugter von ca. 0,25 cm² auf. Es ist jedoch auch möglich, noch kleinere Teilstücke mit einer Größe < 0,1 cm² auszubilden. Besonders kleine Teilstücke sind insbesondere zur Erzielung einer besonders hohen Flexibilität erwünscht. Größere Teilstücke sind bevorzugt, wenn eine höhere mechanische Stabilität gewünscht wird. In Ausführungsformen mit besonders kleinteiligen Teilstücken im innenliegenden Bereich der schichtförmigen Biomatrices und größeren Teilstücken im Randbereich werden bevorzugt innenliegende Teilstücke mit einer Größe bis ca. 0,5 cm² mit randseitig angeordneten Teilstücken mit einer Größe ≥ 1 cm² kombiniert.

Die die Teilstücke (2) miteinander verbindenden Perforationen (3) stellen Perforationen im üblichen Sinne dar und können prinzipiell mit üblichen Perforationsverfahren in der schichtförmigen Biomatrix ausgebildet werden. Bevorzugt werden die Perforationen durch Schneiden oder Stanzen in der Biomatrix ausgebildet. Schneiden kann beispielsweise durch geeignete Messer oder Schneidwerkzeuge wie z.B. Rollenstanzen oder auch mittels Laserschneiden erfolgen. Dabei ist prinzipiell darauf zu achten, dass der Schnitt nicht zu einer vollständigen Trennung der Teilstücke führt, sondern so geführt wird, dass zwischen den Teilstücken genug Matrix-Material z.B. in Form von Fasern oder Materialstegen bestehen bleibt, um die Teilstücke miteinander zu verbinden.

Entsprechend wird durch die erfindungsgemäßen Perforationen eine Art Schwächungslinie oder Sollbruchstelle zwischen den durch diese Perforationen miteinander verbundenen Teilstücken gebildet. Insbesondere umfassen Perforationen somit im Sinne der vorliegenden Erfindungen auch an den Außenkanten der Biomatrix-Teilstücke ausgebildete Fasern oder Materialstege oder sozusagen Diomatrix-Matorialausdünnungen oder - schwächungen, durch die die Teilstücke derart miteinander verbunden sind, daß deren Gesamtheit die Form der schichtförmigen, aus den zusammengesetzten Teilstücke gebildeten Biomatrix bedingen.

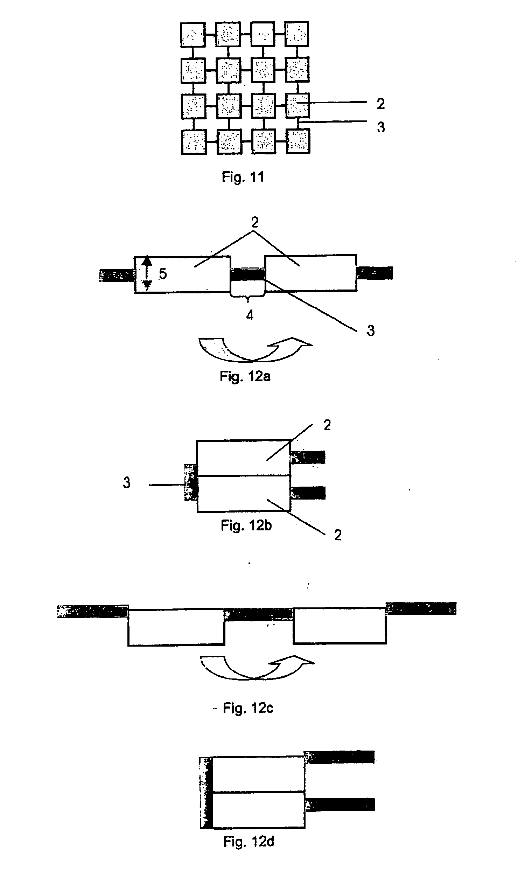

Neben der Herstellung von Perforationen durch einfaches Einschneiden der Biomatrices sind insbesondere auch Perforationen mit einer gezielten Breite bevorzugt. Die Spaltbreite zwischen den Teilstücken (2) kann dabei von einem einfachen Einschnitt (quasi ohne Abstand) bis zur sechsfachen, vorzugsweise vierfachen, besonders bevorzugt zweifachen Schichtdicke der schichtförmigen Biomatrix reichen. Bei einer Spaltbreite entsprechend mindestens der zweifachen Schichtdicke der Biomatrix bzw. der Teilstücke wird eine Verdreh- bzw. Verdrillbarkeit der einzelnen Elemente zueinander um 180° ermöglicht, ohne daß die Teilstücke selbst gebogen oder geknickt werden müssen oder die Perforationen, die die Teilstücke verbinden, aufgetrennt werden. Dadurch kann die Flexibilität und Anpassung der entsprechend perforierten Biomatrix an unregelmäßige Oberflächenstrukturen und insbesondere Hohlräume wie Wundkavitäten weiter verbessert werden. Eine schematische Darstellung einer derartig verbreiterten Perforation bzw. eines derartigen Spalts zwischen den die schichtförmige Biomatrix bildenden Teilstücken (2) zeigt Figur 11. Figur 12a zeigt eine schematische Seitenansicht zweier miteinander verbundener Teilstücke (2), die mittig miteinander verbunden sind. Entspricht die zwischen den Teilstücken (2) gebildete Spaltbreite der Schichtdicke der Biomatrix und damit der Schichtdicke der Teilstücke (2), können die Teilstücke um 180° gedreht und quasi aufeinander positioniert werden, ohne dass die Perforation aufgetrennt wird, wie schematisch in Figur 12b dargestellt. Figur 12c zeigt eine schematische Seitenansicht zweier miteinander verbundener Teilstücke (2), die randseitig miteinander verbunden sind. Entspricht die zwischen den Teilstücken (2) gebildete Spaltbreite der zweifachen Schichtdicke der Biomatrix und damit der zweifachen Schichtdicke der Teilstücke (2), können die Teilstücke um 180° gedreht und quasi aufeinander positioniert werden, ohne dass die Perforation aufgetrennt wird, wie schematisch in Figur 12d dargestellt.

Erfindungsgemäß weist die schichtförmige Biomatrix, und damit auch die die schichtförmige Biomatrix bildenden Teilstücke, eine Dicke (definiert als die kürzeste Entfernung zweier Punkte d.h. Schichtdicke) von maximal 8 mm auf. Bevorzugt weist die schichtförmige Biomatrix eine Schichtdicke bis 5 mm, bevorzugter bis 3 mm auf.

Ein entsprechend großer Abstand zwischen den einzelnen Teilstücken (2) ermöglicht ferner eine leichtere Handhabung bei der Abtrennung kleinerer Segmente oder gar einzelner Teilstücke (2). Durch einen breiteren Perforationsspalt entsteht mehr Platz zum Greifen der herauszutrennenden Einheiten bzw. Segmente bzw. zum Ansetzen der gewählten Schneidwerkzeuge.

Durch die Perforationen (3), durch die die Teilstücke (2) miteinander verbunden sind, ist es möglich, entlang der Perforationen kleinere Segmente variabler Größe aus der schichtförmigen Biomatrix ab- oder herauszutrennen. Dabei wird die Größe solcher heraustrennbaren kleineren Segmente im wesentlichen von der gewählten Perforation bestimmt. Als kleinste heraustrennbare Untereinheit kann ein einzelnes der Teilstücke (2) erhalten werden. Als größte heraus- bzw. abtrennbare Untereinheit kann danach eine um ein einzelnes Teilstück (2) reduzierte schichtförmige Biomatrix betrachtet werden. Grundsätzlich ist die Auftrennung entlang jeder der ausgebildeten Perforationen (3) möglich, womit prinzipiell eine Heraustrennung von Untersegmenten mit hoher individueller flächiger Ausgestaltung möglich ist. Beispielsweise ist es möglich, kleinere Teile variabler Form von der schichtförmigen Biomatrix (1) abzutrennen und separat als kleinere, individuell geformte Auflage zu verwenden. Dies ist insbesondere vorteilhaft zur Anpassung der schichtförmigen Biomatrices an unregelmäßig ausgebildete, flächige Behandlungsareale bzw. Wundränder. Außerdem ist denkbar, aus der schichtförmigen Biomatrix (1) mindestens ein innenliegendes Teilsegment herauszutrennen, um dadurch eine oder mehrere Öffnungen variabler Form und Größe sowie ggf. variabler Anordnung in der schichtförmigen Biomatrix (1) auszubilden und die derart mit der oder den variablen Öffnungen oder Ausnehmungen versehene schichtförmige Biomatrix (1) als Auflage im Sinne der Erfindung zu verwenden.

Prinzipiell umfasst das Abtrennen von Teilsegmenten von der schichtförmigen Biomatrix (1) im Sinne der vorliegenden Erfindung auch das Auftrennen einer oder mehrerer Perforationen wobei die Teilsegmente oder Teilstücke durch eine weitere Perforation weiterhin mit der schichtförmigen Biomatrix (1) verbunden bleiben, so dass durch die aufgetrennte Perforation keine vollständige Herauslösung bzw. Abtrennung der Teilsegmente oder Teilstücke (2) aus der Biomatrix erfolgt, sondern eine Art Einschnitt oder Schlitzung der schichtförmigen Biomatrix (1) erfolgt. Derartige Einschnitte oder Schlitzungen können wiederum in ihrer Länge, Größe oder Ausgestaltung (z.B. linear, kreuz-, sternförmig etc.) variiert werden.

Damit betrifft die vorliegende Erfindung insbesondere auch eine schichtförmige Biomatrix (1), die aus regelmäßig geometrisch geformten, gleichmäßig angeordneten Teilstücken (2), die durch kontinuierliche, über die Fläche der schichtförmigen Biomatrix verlaufende Perforationen (3) abtrennbar miteinander verbunden sind, gebildet wird und die durch Abtrennung von einem oder mehrerer Teilsegmente variabler Größe und Form, die aus mindestens einem der regelmäßig geformten Teilstücke (2) gebildet werden, entlang der Perforationen (3) hinsichtlich ihrer Größe, Form und/oder Ausgestaltung variierbar ist.

Das Durch- oder Auftrennen der Perforationen (3) und damit die Anpassung der Ausgestaltung der Biomatrix (1), wie insbesondere das Zerlegen der erfindungsgemäßen schichtförmigen Biomatrix (1) in kleinere Teilsegmente oder gar in die einzelnen Teilstücke (2) kann beispielsweise durch einfaches Reißen, z.B. manuell, entlang des gewählten Perforationsverlaufs unter Ausbildung der gewünschten Form des herauszutrennenden Teilsegments erfolgen. Alternativ können kleinere Segmente oder die Teilstücke (2) auch unter Zuhilfenahme geeigneter Hilfsmittel, wie einer Schere oder einem Messer, abgetrennt werden. Augrund der durch die Perforationen gebildeten Sollbruchstellen können dabei vorteilhafterweise auch wenig scharfe und damit vergleichsweise ungefährliche Schneidwerkzeuge verwendet werden.

Prinzipiell können die aus der Biomatrix (1) durch Abtrennung ausgebildeten Teilsegmente gleichzeitig oder aber auch separat, beispielsweise zu einem späteren Zeitpunkt, verwendet werden.

Die Figuren 1 bis 12 erläutern beispielhaft den Gegenstand der vorliegenden Erfindung und stellen verschiedene mögliche Ausgestaltungen und Ausführungsformen der erfindungsgemäßen schichtförmigen Biomatrix dar. Die darin verwendeten Bezugszeichen beziehen sich auf die vorstehenden Erläuterungen:
Figur 1 zeigt eine beispielhafte Ausführungsform der erfindungsgemäßen schichtförmigen Biomatrix, worin die durch die kontinuierlichen, durchgehenden Perforationen verbundenen Teilstücke quadratisch ausgebildet sind.
Figur 2 zeigt eine beispielhafte Ausführungsform der erfindungsgemäßen schichtförmigen Biomatrix, worin die Abstände zwischen den parallel angeordneten kontinuierlichen, durchgehenden Perforationen unterschiedlich sind, wodurch Teilstücke unterschiedlicher Größe gebildet werden.
Figur 3 zeigt eine beispielhafte Ausführungsform der erfindungsgemäßen schichtförmigen Biomatrix, worin die durch die kontinuierlichen, durchgehenden Perforationen verbundenen Teilstücke als Parallelogramme ausgebildet sind.
Figur 4 zeigt eine beispielhafte Ausführungsform der erfindungsgemäßen schichtförmigen Biomatrix, worin die durch die kontinuierlichen, durchgehenden Perforationen verbundenen Teilstücke als Dreiecke ausgebildet sind.
Figur 5 zeigt eine beispielhafte Ausführungsform der erfindungsgemäßen schichtförmigen Biomatrix, worin die Perforationen derart ausgebildet sind, dass zueinander versetzt angeordnete rechteckige Teilstücke gebildet werden, wodurch ein Muster entsteht, das mit einem Mauerverband nach Art eines Läufer- oder Binderverbandes vergleichbar ist.
Figur 6 zeigt eine beispielhafte Ausführungsform der erfindungsgemäßen schichtförmigen Biomatrix, worin die durch die kontinuierlichen, durchgehenden Perforationen verbundenen Teilstücke trapezförmig ausgebildet sind.
Figur 7 zeigt eine beispielhafte Ausführungsform der erfindungsgemäßen schichtförmigen Biomatrix, worin die durch die kontinuierlichen, durchgehenden Perforationen verbundenen Teilstücke wabenförmig ausgebildet sind.
Figur 8 zeigt eine beispielhafte Ausführungsform der erfindungsgemäßen schichtförmigen Biomatrix, worin die durch die kontinuierlichen, durchgehenden Perforationen verbundenen Teilstücke kreisförmig ausgebildet sind.
Figur 9 zeigt eine beispielhafte Ausführungsform der erfindungsgemäßen schichtförmigen Biomatrix, worin die durch die kontinuierlichen, durchgehenden Perforationen verbundenen Teilstücke in unterschiedlichen geometrischen Formen ausgebildet sind, so dass gleichmäßig angeordnete Areale (6a) mit als Dreiecke ausgebildeten Teilstücken und ein weiteres Areal (6b) mit quadratisch ausgebildeten Teilstücken gebildet werden.
Figur 10 zeigt eine beispielhafte Ausführungsform der erfindungsgemäßen schichtförmigen Biomatrix, worin die durch die kontinuierlichen, durchgehenden Perforationen verbundenen Teilstücke in unterschiedlichen geometrischen Formen ausgebildet sind, indem auf einer insgesamt kreisförmigen Biomatrix weitere kreisförmige Perforationen kleinerer Radien sowie sich im Mittelpunkt schneidende durchgehende Perforationen angebracht werden, wodurch Areale (6a, 6b) mit im wesentlichen tortenstückartigen bzw. bogenförmigen Teilstücken gebildet werden.
Figur 11 zeigt eine schematische Darstellung einer verbreiterten Perforation bzw. einer als Spalt ausgebildeten Perforation (3) zwischen den die schichtförmige Biomatrix bildenden Teilstücken (2).
Figur 12a zeigt eine schematische Seitenansicht zweier miteinander verbundener Teilstücke (2), die mittig miteinander durch eine Perforation (3) verbunden sind und worin die Perforation einen Spalt (4) zwischen den Teilstücken (2) bildet, und worin der Spalt (4) eine der Schichtdicke (5) der Biomatrix und damit der Schichtdicke (5) der Teilstücke (2) entsprechende Breite aufweist.
Figur 12b zeigt eine schematische Seitenansicht, worin zwei der durch eine Perforation (3) miteinander verbundenen Teilstücke (2) um 180° gegeneinander verdreht wurden, ohne dass die Perforation (3) aufgetrennt ist.
Figur 12c zeigt eine der Figur 10a entsprechende Darstellung, worin die Teilstücke (2) randseitig durch die Perforation (3) miteinander verbunden sind.
Figur 12d zeigt eine der Figur 10b entsprechende Darstellung, worin die Teilstücke (2) randseitig durch die Perforation (3) miteinander verbunden sind.

In den Figuren 1 bis 12 weisen die Bezugszeichen folgende Bedeutung auf:
- 1: schichtförmige Biomatrix
- 2: Teilstück
- 3: Perforation
- 3a, 3b: sich schneidende Perforationen
- 4: als Spalt ausgebildete Perforation
- 5: Dicke der schichtförmigen Biomatrix bzw. der Teilstücke
- 6a, 6b: unterschiedliche Areale der schichtförmigen Biomatrix mit jeweils regelmäßig geformten Teilstücken

Im Folgenden wird der strukturelle Aufbau der erfindungsgemäßen schichtförmigen Biomatrix näher beschrieben.

Die schichtförmige Biomatrix umfasst erfindungsgemäß ein Trägermaterial, welches für die erfindungsgemäße Anwendung als Haut- bzw. Wundauflage, ggf. auch zur Applikation von Wirk- und Pflegesubstanzen, zur Aufnahme von Flüssigkeiten geeignet ist.

Dabei kann die schichtförmige Biomatrix entweder vollständig aus dem Trägermaterial gebildet sein oder zu einem überwiegenden Teil daraus bestehen. Beispielsweise kann die schichtförmige Biomatrix auf einem Trägermaterial basieren, dem zusätzliche Wirk- und/oder Hilfsstoffe zugesetzt wurden oder die auf einem Trägermaterial basierende schichtförmige Biomatrix kann außerdem eine zusätzliche Beschichtung aufweisen.

Die Anwendung der erfindungsgemäßen schichtförmigen Biomatrices erfolgt bevorzugt in trockener Form. Es ist jedoch auch eine Anwendung in angefeuchteter bzw. vorbefeuchteter Form möglich, sowie eine Anwendung, worin eine Befeuchtung im Verlauf der Anwendung bzw. Behandlung vorgenommen wird.

Weiterhin muss das Trägermaterial so ausgewählt werden, dass dieses eine ausreichende Stabilität aufweist, um durch Zuschneiden und Perforieren in die erfindungsgemäßen schichtförmigen Biomatrices überführt werden können. Darüber hinaus müssen die erfindungsgemäßen schichtförmigen Biomatrices eine ausreichende mechanische Stabilität besitzen, um auch während der Verwendung bzw. im Verlauf der Applikation, insbesondere beim Trennen perforierter Segmente, sowie beim Auftragen und Anmodellieren der schichtförmigen Biomatrix bzw. einzelner abgetrennter Segmente davon auf die zu behandelnde Körperregion, jeweils sowohl im trockenen als auch im angefeuchteten Zustand, formstabil zu bleiben und insbesondere nicht zu reißen.

Erfindungsgemäß handelt es sich um eine schichtförmige Biomatrix aus einem Trägermaterial, umfassend mindestens ein Polymer aus der Gruppe der strukturbildenden hydrophilen natürlichen Polymere und/oder der bioabsorbierbaren synthetischen und/oder modifizierten natürlichen Polymere.

Vorzugsweise wird das Trägermaterial aus der Gruppe der natürlichen hydrophilen, d.h. mit Wasser benetzbaren Materialien gewählt. Bevorzugt ist es ein sogenannter Strukturbildner oder ein strukturbildendes Hydrokolloid, also ein teilweise wasserlösliches oder wasserquellbares natürliches, strukturbildendes Polymer. Insbesondere bevorzugt sind strukturbildende Hydrokolloide aus den Gruppen der Proteine, der Polysaccharide und/oder der Glucosaminoglykane.

Besonders bevorzugt wird das Trägermaterial der schichtförmigen Biomatrix aus der Gruppe der Proteine ausgewählt wie z. B. Collagen, beispielsweise lösliches oder unlösliches, fibrilläres, tierisches oder pflanzliches Collagen, oder Gelatine, Elastin, Keratin, Fibroin, Albumine, Globuline wie Lactoglobulin, Milchproteine wie Casein. Dabei ist Collagen ganz besonders bevorzugt, ggf. auch in Mischung mit weiteren fibrillären Proteinen oder in Mischung mit Gelatine oder besonders bevorzugt in Mischung mit Elastin. Bei Trägermaterialien auf Basis von Collagen handelt es sich bevorzugt um solche, die nach aus dem Stand der Technik und z. B. aus der DE 4028622 oder aus der DE 10350654 bekannten Verfahren aufgearbeitet und hergestellt werden. Die erfindungsgemäß bevorzugten Collagenträgermaterialien zeichnen sich insbesondere durch herausragende Hydratationseigenschaften und eine gute Flüssigkeitsaufnahmekapazität bzw. Saugfähigkeit, ein Aspekt der insbesondere zur Aufnahme großer Flüssigkeitsmengen z. B. bei stark blutenden Wunden oder bei Wunden mit einem hohen Maß an ausgesonderter Wundflüssigkeit vorteilhaft ist, sowie durch deren antiirritative und hautberuhigende Eigenschaften aus was vorteilhaft in der erfindungsgemäßen Anwendung in der Hautbehandlung ist. Aufgrund der strukturellen Ähnlichkeit mit der menschlichen Haut und menschlichem Gewebe werden bevorzugt Collagenarten ausgewählt, die in Haut und Gewebe auftreten, insbesondere Collagen des Typs I, III und V. Dadurch bedingt sich die besonders gute Verträglichkeit und Biokompatibilität solcher erfindungsgemäßer Collagen-Trägermaterialien. Die hiermit erhältlichen Mittel sind darüber hinaus im Körper biologisch abbaubar und können bei Verbleib in einer Wunde, z.B. bei Verwendung als Implantat, auf natürliche Weise verstoffwechselt werden. Dadurch sind derartige Trägermaterialien besonders für die Herstellung von Wundbehandlungsmitteln oder Hämostyptika zur Verwendung als Implantat geeignet. Das erfindungsgemäß verwendete Collagen-Trägermaterial wird bevorzugt aus Collagenquellen bovinen, equinen und porcinen Ursprungs gewonnen. Ganz besonders bevorzugt ist bovines Collagen. Das Collagen kann nach üblichen Verfahren aus den üblichen Quellen wie Häuten oder Sehnen gewonnen werden.

Darüber hinaus können auch Collagenmaterialien verwendet werden, welche einer Vernetzungsreaktion unterworfen wurden. In diesem Fall ist eine thermische Vernetzung, die so genannte Dehydrothermalvernetzung, bevorzugt. Ferner ist die Vernetzung mit chemischen Vernetzern möglich. Hierzu zählen insbesondere Aldehyde, wie Glutaraldehyd; Carbodiimide, wie EDC; Isocyanate; Epoxide oder Imidazole, wobei das Epoxid aus der Gruppe der chemischen Vernetzer besonders bevorzugt ist.

Ebenfalls bevorzugte Trägermaterialien, die aus der Gruppe der Polysaccharide ausgewählt werden, schließen beispielsweise Homoglykane oder Heteroglykane, wie zum Beispiel Alginate, besonders Natriumalginat oder Calciumalginat oder Mischungen davon, Carrageen, Pektine, Tragant, Cuar-Cummi, Johannisbrotkernmehl, Agar-Agar, GummiArabikum, Xanthan, natürliche und modifizierte Stärken, Dextrane, Dextrin, Maltodextrine, Chitosan, Glucane wie β-1,3-Glucan oder β-1,4-Glucan, Cellulose etc. ein. Besonders bevorzugte Polysaccharide sind Alginate, insbesondere Natrium-Alginate und Calcium-Alginate oder Mischungen davon.

Die Gruppe der Trägermaterialien, die aus der Gruppe der Polysaccharide ausgewählt sind, umfasst ebenfalls solche Materialien, die einer Vernetzung unterworfen wurden. Insbesondere schließen vernetzte Polysaccharide mit Calcium-Ionen vernetzte Alginate ein.

Glucosaminoglycane (Mucopolysaccharide) schließen beispielsweise ein Hyaluronsäure, Chondroitinsulfat, Dermatansulfat, Keratansulfat, Heparansulfat, Heparin etc. Besonders bevorzugt ist Hyaluronsäure.

Weiterhin können auch Trägermaterialien verwendet werden, die aus der Gruppe der bioabsorbierbaren synthetischen bzw. modifizierten natürlichen Polymere, umfassend z.B. Polylactide oder Polymilchsäuren (PLA), Polyglycolsäure (PGA), Polycaprolactone (PCL), Polydioxanone (PDO), Polylactid-co-Glycolide (PLGA), Polytrimethylencarbonat etc. ausgewählt sind.

Es können auch Mischungen aus mindestens zwei verschiedenen der oben genannten Trägermaterialien verwendet werden. Dabei sind insbesondere Mischungen z. B. aus Collagen und Gelatine oder aus Collagen und Alginaten oder aus Alginaten und Hyaluronsäure, ggf. auch in Mischung mit weiteren der genannten Trägermaterialien, sowie Mischungen der vorstehend genannten bioabsorbierbaren synthetischen bzw. modifizierten natürlichen Polymere mit Collagen oder Alginat bevorzugt.

Grundsätzlich können die Trägermaterialien der erfindungsgemäßen schichtförmigen Biomatrix auch geringe Mengen synthetischer und/oder semi-synthetischer und/oder modifizierter natürlicher Polymere enthalten, wie z. B. solche die aus der Gruppe umfassend beispielsweise Celluloseether, Polyvinylalkohol, Polyvinylpyrrolidon, synthetische Cellulosederivate, wie Methylcellulose, Carboxycellulose, Carboxymethylcellulose wie z. B. Natrium-Carboxymethylcellulose, Celluloseester, Celluloseether wie Hydroxypropylcellulose, kationisierte Cellulosen oder kationisierte Stärken etc., Polyacrylsäure, Polymethacrylsäure, Poly(methylmethacrylat) (PMMA), Polymethacrylat (PMA), Polyethylenglykole, Polyurethane, Polyharnstoffverbindungen etc. und Mischungen davon ausgewählt sind. Ein bevorzugtes synthetisches Polymer ist Polyacrylat bzw. Polyacrylsäure, welches ganz besonders bevorzugt z.B. in Mischung mit Trägermaterialien die aus den Alginaten ausgewählt sind, in den schichtförmigen Biomatrices enthalten sein kann. Bevorzugte semi-synthetische bzw. modifizierte natürliche Polymere sind solche umfassend Cellulose, Carboxymethylcellulose, kationisierte Cellulosen oder kationisierte Stärken.

Der Anteil solcher synthetischer und/oder semi-synthetischer und/oder modifizierter natürlicher Polymere in den erfindungsgemäßen schichtförmigen Biomatrices liegt dabei in der Regel unter 40 Gew.-%, bevorzugter unter 30 Gew.-%, noch bevorzugter unter 20 Gew.-%, jeweils bezogen auf das Gesamtgewicht der trockenen schichtförmigen Biomatrix.

Ganz besonders bevorzugt sind jedoch solche schichtförmigen Biomatrices, die keine synthetischen Trägermaterialien enthalten, wobei hier nicht die vorstehend beschriebenen bioabsorbierbaren synthetischen bzw. modifizierten natürlichen polymeren Trägermaterialien gemeint sind.

Der Anteil der vorstehend genannten bioabsorbierbaren synthetischen bzw. modifizierten natürlichen Polymere in den erfindungsgemäßen schichtförmigen Biomatrices liegt in der Regel unter 70 Gew.-%, bevorzugter unter 60 Gew.-%, noch bevorzugter unter 50 Gew.-%, jeweils bezogen auf das Gesamtgewicht der trockenen schichtförmigen Biomatrix.

Erfindungsgemäß ist die Verwendung von vernetzten Trägermaterialien zur Herstellung der erfindungsgemäßen schichtförmigen Biomatrix besonders bevorzugt, da vernetzte Materialien über eine besonders hohe mechanische Stabilität verfügen. Dadurch sind vernetzte Trägermaterialien umfassende schichtförmige Biomatrices besonders gut geeignet, um die erfindungsgemäßen Perforationen anzubringen. Weiterhin sind derart mechanisch stabilisierte erfindungsgemäße schichtförmige Biomatrices auch besonders gut für die erfindungsgemäße Anwendung und Applikation geeignet, da sie einerseits über eine gute Stabilität aufweisen, so daß beim Trennen der Perforationen die Abtrennung der Teilsegmente der gewünschten Form und Größe ohne eine Beschädigung weiterer Teile der schichtförmigen Biomatrix oder der herauszutrennenden Teile und andererseits die Applikation und Modellierbarkeit der schichtförmigen Biomatrix oder der davon abgetrennten Teile auf der zu behandelnden Körperregion erleichtert wird.

Weiterhin können die schichtförmigen Biomatrices laminiert sein bzw. die schichtförmigen Biomatrices können durch Trägermaterialien in Form mehrschichtiger, miteinander verbundener Lagen gebildet werden. Als Laminate können übliche, aus dem Stand der Technik bekannte Materialien wie z.B. Fasern, Vliese, Netze, Filme oder Folien aus geeigneten Materialien wie z.B. Rayon, Cellulose, Polyethylen (PE) oder Polyurethan (PU) oder anderen synthetischen oder semi-synthetischen Polymeren / Copolymeren verwendet werden, die mit herkömmlichen Methoden, z.B. durch Kleben, Heißlaminieren, Vernetzen etc. mit den Trägermaterialien im Sinne der vorliegenden Erfindung fest verbunden werden können. Eine derartige Laminierung ist besonders geeignet, um die mechanische Stabilität der Trägermaterialien zur Herstellung der erfindungsgemäßen schichtförmigen Biomatrices zu erhöhen. Bevorzugt wird dabei die Laminierung vor dem Anbringen der erfindungsgemäßen Perforationen auf das Trägermaterial aufgebracht.

Trägermaterialien, die zur Herstellung der erfindungsgemäßen schichtförmigen Biomatrix geeignet sind, können nach üblichen Verfahren, wie beispielsweise in der DE 4028622, DE 10350654, der WO 04/104076, der WO 05/113656 oder der WO 08/020066 der Anmelderin beschrieben, hergestellt werden.

Die Trägermaterialien der erfindungsgemäßen schichtförmigen Biomatrix weisen eine gute Biokompatibilität auf und sind insbesondere haut- und schleimhautverträglich und weisen weder bei der Anwendung auf intakter Haut noch beim Einbringen in eine der unteren Hautschichten z.B. in Wunden, die eine Verletzung oder Zerstörung des natürlichen Hautaufbaus aufweisen, ein toxikologisches Potential auf. Auch rufen die erfindungsgemäß zu verwendenden Polymere bei der Applikation keinerlei Irritationswirkungen oder andere Unverträglichkeitsreaktionen hervor. Sie sind pharmakologisch völlig unbedenklich und somit optimal geeignet als Polymermaterial für die erfindungsgemäßen kosmetischen und pharmazeutischen dermalen Verwendungen.

Die erfindungsgemäße schichtförmige Biomatrix kann darüber hinaus mindestens einen Wirkstoff umfassen. Wirkstoffe schließen insbesondere kosmetische oder therapeutische bzw. pharmazeutische, für die äußere Anwendung geeignete Wirkstoffe ein. Dementsprechend handelt es sich bei solchen erfindungsgemäßen Zusammensetzungen bevorzugt um kosmetische oder therapeutische Mittel.

Kosmetische Mittel bzw. unter Verwendung kosmetischer Wirkstoffe hergestellte Mittel im Sinne der Erfindung sind im wesentlichen Mittel im Sinne des Lebensmittel-, Bedarfsgegenstände- und Futtermittelgesetzbuches (LFGB), d.h., Stoffe oder Zubereitungen aus Stoffen, die dazu bestimmt sind, äußerlich am Menschen zur Reinigung, Pflege, oder zur Beeinflussung des Aussehens oder des Körpergeruchs, oder zur Vermittlung von Geruchseindrücken angewendet zu werden, es sei denn, dass sie überwiegend dazu bestimmt sind, Krankheiten, Leiden, Körperschäden oder krankhafte Beschwerden zu lindern oder zu beseitigen. In diesem Sinne handelt es sich bei den erfindungsgemäß verwendeten kosmetischen Formkörpern beispielsweise um Badepräparate, Hautwasch- und -reinigungsmittel, Hautpflegemittel, insbesondere Gesichtshautpflegemittel, Augenkosmetika, Lippenpflegemittel, Nagelpflegemittel, Fußpflegemittel, Haarpflegemittel, insbesondere Haarwaschmittel, Haarkonditionierungsmittel, Haarweichspüler etc., Lichtschutzmittel, Hautbräunungs- und -aufhellungsmittel, Depigmentierungsmittel, Deodorants, Antihydrotika, Haarentfernungsmittel, Insektenrepellents etc. oder derartige Mittel in Kombination.

Beispiele kosmetisch gegebenenfalls auch z.B. dermatologischer, therapeutisch wirksamer Verbindungen schließen ein: Antiaknemittel, antimikrobielle Mittel, Antitranspirationsmittel, adstringierende Mittel, desodorierende Mittel, Enthaarungsmittel, Konditionierungsmittel für die Haut, hautglättende Mittel, Mittel zur Steigerung der Hauthydratation wie z. B. Glycerin oder Harnstoff, Sonnenschutzmittel, Keratolytika, Radikalfänger für freie Radikale, Antiseborrhöika, Antischuppenmittel, antiseptische Wirkstoffe, Wirkstoffe zur Behandlung der Anzeichen der Hautalterung und/oder Mittel, die die Differenzierung und/oder Proliferation und/oder Pigmentierung der Haut modulieren, Vitamine wie Vitamin C (Ascorbinsäure) und ihre Derivate, wie beispielsweise Glycoside wie Ascorbylglucosid, oder Ester der Ascorbinsäure wie Natrium- oder Magnesium-Ascorbylphosphat oder Ascorbylpalmitat und -stearat L-Ascorbinsäurephosphatester, Alkalimetallsalze wie Natrium- und Kaliumsalze von L-Ascorbinsäurephosphatestern; Erdalkalimetallsalze wie Magnesium- und Calciumsalze von L-Ascorbinsäurephosphatestern; trivalente Metallsalze wie Aluminiumsalze von L-Ascorbinsäurephosphatestern; Alkalimetallsalze von L-Ascorbinsäuresulfatestern wie Natrium- und Kaliumsalze von L-Ascorbinsäuresulfatestern; Erdalkalimetallsalze wie Magnesium- und Calciumsalze von L-Ascorbinsäuresulfatestern; trivalente Metallsalze wie Aluminiumsalze von L-Ascorbinsäuresulfatestern; Alkalimetallsalze wie Natrium- und Kaliumsalze von L-Ascorbinsäureestern; Erdalkalimetallsalze wie Magnesium- und Calciumsalze von L-Ascorbinsäureestern; und trivalente Metallsalze wie Aluminiumsalze von L-Ascorbinsäureestern.

Wirkstoffe mit reizender Nebenwirkung, wie alpha-Hydroxysäuren, β -Hydroxysäuren, alpha-Ketosäuren, β-Ketosäuren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, mehrfach ungesättigte Fettsäuren, essentielle Fettsäuren (z.B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, oder dekorative Stoffe wie Pigmente oder Farbstoffe und -partikel für Foundations, Make-up Formulierungen, und andere Mittel zur kosmetischen Verschönerung und farblichen Gestaltung von Augen-, Lippen-, Gesicht etc. sowie abrasive Mittel.

Weiterhin können Pflanzenwirkstoffextrakte bzw. daraus gewonnene Auszüge oder Einzelstoffe erwähnt werden. Allgemein wird der Pflanzenwirkstoffextrakt in der Regel ausgewählt aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskautöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl); Immunstimulantien: Echinacea purpurea (z.B.alkoholische Auszüge, Frischpflanzensaft, Preßsaft), Eleutherokokkus senticosus; Alkaloide: Koffein, Teein, Theobromin, Rauwolfia (z.B. Prajmalin), Immergrün (z.B.Vincamin); weitere Phytopharmaka: Aloe, Roßkastanie (z.B. Aescin), Knoblauch (z.B. Knoblauchöl), Ananas (z.B. Bromelaine), Ginseng (z.B. Ginseno-side), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzslextrakte, Palmenextrakt, Galphimia (z.B.Urtinktur), Mistel (z.B. wässrig-ethanol. Auszug), Phytosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordica charantia, und Aloe Vera Extrakte.

Im Unterschied zu den vorstehend beschriebenen im wesentlichen in der Kosmetik verwendeten Wirkstoffen handelt es sich bei den therapeutischen Wirkstoffen (Arzneimitteln) um solche, die im Sinne des Arzneimittelgesetzes unter anderem dazu bestimmt sind, Krankheiten, Leiden, Körperschäden oder krankhafte Beschwerden zu heilen, zu lindern oder zu verhüten. Erfindungsgemäß sind insbesondere solche Mittel bzw. Wirkstoffe geeignet, die für die äußere oder transdermale Anwendung, insbesondere im Bereich der Wundbehandlung und -heilung sowie im Bereich der Behandlung von Brandverletzungen, insbesondere zur Erstversorgung von Brandwunden bestimmt sind.

Bei Wirkstoffen für eine dermale oder transdermale Anwendung handelt es sich insbesondere um hautaktive aber auch um transdermale Wirkstoffe. Sie schließen beispielsweise ein: Mittel zur Behandlung von Brandverletzungen, Mittel zur Behandlung von Hautkrankheiten, äußerlich anwendbare Analgetika, z. B. Dextropropoxyphen, Pentazocin, Pethidin, Buprenorphin; Antirheumatika/Antiphlogistika (NSAR), z. B. Indometacin, Diclofenac, Naproxen, Ketoprofen, Ibuprofen, Flurbiprofen, Salicylsäure und -derivate wie Acetylsalicylsäure, Oxicame; Steroidhormone, z. B. Betamethason, Dexamethason, Methylprednisolon, Ethinylestradiol, Medroergotamin, Dihydroergotoxin; Gichtmittel, z. B. Benzbromaron, Allopurinol; Dermatika Externa, Antihistaminika, Antibiotika einschließlich antibakterielle Mittel wie z.B. kolloidales Silber und Silbersalze, Antimykotika, Peptidarzneistoffe, antivirale Wirkstoffe, entzündungshemmende Wirkstoffe, juckreizstillende Wirkstoffe, anästhesierende Wirkstoffe, z. B. Benzocain, Corticoide, Aknemittel, antiparasitäre Wirkstoffe; äußerlich anwendbare Hormone; Venentherapeutika; Immunsuppresiva etc. alle für die dermale oder transdermale Anwendung.

Bevorzugte therapeutische Mittel für die dermale und transdermale Anwendung sind Mittel zur Behandlung von Hautkrankheiten, wie der Neurodermitis, der atopischen Dermatitis etc., und Anti-Herpesmittel, sowie insbesondere solche, die im Bereich der Wundbehandlung, insbesondere zur Behandlung von chronischen Wunden, Dekubitus, Ulcus cruris, diabetischem Fußsyndrom etc. eingesetzt werden wie beispielsweise Analgetika, z.B. Immunsuppressiva, Hormone, anästhesierende Wirkstoffe, antiparasitäre, fungizide bzw. antimykotische und antibakterielle Wirkstoffe wie insbesondere Silber-haltige Wirkstoffe wie z. B. Silbernitrat, Silberchlorid, Silberjodid oder weitere aus dem Stand der Technik bekannte Silber-haltige Wundbehandlungsstoffe, Wirkstoffe zur Unterstützung und Regulierung des Wundmilieus wie insbesondere Elektrolyte, Kieselerde, Mineralstoffe und Spurenelemente wie z. B. Kalium, Magnesium, Calcium, Selen, Iod etc., Wirkstoffe zur Erzielung eines Wunddebridements wie z. B. Kollagenasen oder andere, im Stand der Technik bekannte, geeignete proteolytischen Enzyme sowie Wirkstoffe zur Unterstützung der Wundheilung wie z.B. Wachstumsfaktoren, Enzyminhibitoren etc..

Weitere bevorzugte Wirkstoffe sind solche, die eine blutstillende oder hämostatische Wirkung aufweisen wie z.B. Thrombin, Fibrinogen oder Cholesterylsulfat (z.B. Natriurn-Cholestorylsulfat) oder Wirkstoffe mit aktivierender Wirkung auf Faktoren und Substanzen der extrinsischen und/oder intrinsischen Gerinnungskaskade wie z. B. Phospholipide, Kaolin, Aprotinin, Faktor- oder Faktoren-Konzentrate, Tissue Factor oder Calcium-Ionen.

Des Weiteren ist denkbar, weitere Wirkstoffe wie Bronchialtherapeutika wie Antiasthmatika, Antitussiva, Mucolytica etc., Antidiabetica wie z. B. Glibenclamid, Hormone, Steroidhormone wie Dexamethason, Herzglycoside wie Digitoxin, Herz- und Kreislauftherapeutika wie z. B. Beta-Blocker, Antiarrhythmika, Antihypertonika, Calcium-Antagonisten etc., Psychopharmaka und Antidepressiva wie z. B. trizyklische Antidepressiva (NSMRI), Serotonin-Wiederaufnahme-Hemmer (SSRI), Noradrenalin-Wiederaufnahme-Hemmer (NRI), Serotonin-Noradrenalin-Wiederaufnahme-Hemmer (SNRI), Monoaminooxidase-Hemmer (MAO-Hemmer) etc., Neuroleptika, Antikonvulsiva oder Antiepileptika, Hypnotika, Sedativa, Anästhetika, Magen-, Darmtherapeutika, Lipidsenker, Analgetika wie z. B. Antimigränemittel, Paracetamol, Salicylsäure und -derivate wie Acetylsalicylsäure, Diclophenac, Ibuprofen, Ketoprofen, Naproxen etc., Antiphlogistika, Vasodilatatoren, Diuretica, Gichtmittel, Zytostatika, Muskelrelaxantien, Kontrazeptiva z. B. in Form von Hormonpflastern, Suchtentwöhnungsmittel in Form von beispielsweise Nicotinpflastern, Pflanzenextrakte, Provitamine wie z. B. Beta-Carotin, Vitamine wie z. B. Vitamin C, A, B, E etc., über eine transdermale Applikation in einer erfindungsgemäßen Zusammensetzung z. B. in Form eines transdermalen Wirkstoff-Patches zu verabreichen.

Auch die Trägermaterialien, insbesondere solche auf Basis proteinogener Polymere wie insbesondere Collagen oder pflanzliche Polymere wie Polysaccharide können gewisse therapeutische Wirkungen aufweisen. So wirkt das bevorzugt verwendete Collagen blutstillend und zeigt einen positiven, unterstützenden Effekt in der Wundheilung. Auch dem bevorzugt verwendeten Hydrokolloid (Natrium-)Alginat wird eine gewisse blutstillende Wirkung nachgesagt. Weiterhin wirkt es in gewissem Ausmaß antiviral. Hyaluronsäure wird eine gewisse Wirkung in der Re-Epithelisierung und als Antioxidans und Feuchtigkeitsspender in der Hautpflege nachgesagt. Sie sind jedoch keine Wirkstoffe im Sinne der Erfindung.

Die erfindungsgemäße schichtförmige Biomatrix kann ferner mindestens einen Hilfsstoff umfassen.

Hilfsstoffe schließen ein: pH-Einstellungsmittel, wie Pufferstoffe, anorganische und organische Säuren oder Basen; Fettsubstanzen, wie Mineralöle, wie Paraffinöle oder Vaselineöle, Siliconöle, Pflanzenöle wie Kokosöl, Süßmandelöl, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamonöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Neutralöl, Squalan, Fettsäureester, Ester von Fettalkoholen wie Triglyceride, und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle (sogenannte kosmetische Öle), wie beispielsweise in der CTFA-Abhandlung, Cosmetic Ingredient Hand-book, 1. Auflg., 1988, The Cosmetic, Toiletry and Fragrance Association, Inc., Washington, erwähnt, oberflächenaktive Mittel neben den oben erwähnten Waschtensiden, wie Dispergiermittel, Netzmittel, Emulgatoren etc.; Füllstoffe; Stabilisatoren; Cosolventien; pharmazeutisch und kosmetisch gebräuchliche oder sonstige Farbstoffe und Pigmente, insbesondere solche, die primär zur farblichen Gestaltung der Hydrogel-Zusammensetzung eingesetzt werden und nicht zur Applikation und farblichen Gestaltung am menschlichen Körper, wie solche Pigmente und Farbstoffe wie die unter der Gruppe der Wirkstoffe aufgeführten dekorativen Farbstoffe; Konservierungsmittel; Weichmacher; Schmiermittel bzw. Gleitmittel; etc.

Erfindungsgemäß bevorzugte Hilfsstoffe sind Fette und Öle. Dabei sind insbesondere kosmetische Öle wie vorstehend aufgezählt, insbesondere Triglyceride, besonders bevorzugt Capryl/Capronsäure-Triglyceride, Squalan oder Jojobaöl bevorzugt.

Generell schließt die Einordnung der vorstehend erwähnten Stoffe in die Kategorie der Hilfstoffe im Rahmen der vorliegenden Erfindung nicht aus, dass diese Hilfsstoffe auch gewisse kosmetische und/oder therapeutische Wirkungen entfalten können, was in besonderem Maß für die genannten bevorzugt eingesetzten kosmetischen Öle gilt.

Die erfindungsgemäße schichtförmige Biomatrix ist erhältlich durch ein Verfahren, umfassend die Schritte:
a) Herstellen einer wässrigen Suspension oder einer Lösung mindestens eines strukturbildenden Polymers;
b) gegebenenfalls Einmischen eines oder mehrerer Wirk- und/oder Hilfsstoffe;
c) Giessen der Mischung in eine geeignete Form;
d) Trocknen der Mischung;
e) Schneiden des aus Schritt d) erhältlichen getrockneten Formkörpers in Schichten, vorzugsweise von maximal 8 mm Dicke; gegebenenfalls
f) Schneiden der Schichten aus Schritt e) in die gewünschte geometrische Ausgestaltung der schichtförmigen Biomatrix (1);
g) Anbringen der kontinuierlichen, durchgehenden Perforationen (3) und dadurch Ausbildung von regelmäßig geformten, gleichmäßig angeordneten Teilstücken (2); sowie gegebenenfalls
h) Sterilisieren und/oder Konfektionieren.

In einer Ausführungsform, umfassend Trägermaterialien aus der Gruppe der o.g. bioabsorbierbaren synthetischen bzw. modifizierten natürlichen Polymere wird in Schritt c) die wässrige Suspension oder Lösung mindestens eines strukturbildenden Polymers gemäß Schritt a) in ein solches, eine Gerüststruktur aufweisendes, bioabsorbierbares Polymer, vorzugsweise in ein Polylactidgerüst oder eines Copolymers davon, eingegossen. Damit umfasst der Begriff "Form" aus Schritt c) definitionsgemäß auch eine durch ein solches bioabsorbierbares Polymer mit Gerüststruktur gebildete Form.

In der Regel ist jedoch unter "Form" eine herkömmliche Volumen- oder Hohlform im üblichen Sinne zu verstehen.

In dem erfindungsgemäßen Verfahren kann die Perforation in Schritt g) mittels Schneiden oder Stanzen erzeugt werden. Dabei ist es insbesondere auch möglich, die Perforationen mittels einer entsprechend geeigneten Anzahl, im gewünschten Abstand parallel angeordneter Perforationswerkzeuge, vorzugsweise Messer oder üblichen Rollenstanzen, durchzuführen.

In einer bevorzugten Ausführungsform werden die Perforationen mittels einer entsprechend geeigneten Anzahl, im gewünschten Abstand parallel angeordneter Perforationswerkzeuge in Form kontinuierlicher, vorzugsweise linearer, zueinander parallel angeordneter durchgehender Perforationen (3a) und, die Perforationen (3a) schneidenden, kontinuierlicher, vorzugsweise linearer, zueinander parallel angeordneter durchgehender Perforationen (3b) angebracht. Dabei kann der Abstand der parallel angeordneten Perforationswerkzeuge jeweils gleich oder verschieden ist. Dabei werden vorzugsweise die kontinuierlichen, zueinander parallel angeordneten durchgehenden Perforationen (3a) bzw. die die Perforationen (3a) schneidenden kontinuierlichen, zueinander parallel angeordneten durchgehenden Perforationen (3b) jeweils in einem Schritt durchgeführt.

Bevorzugt werden die Perforationen (3) so ausgebildet, daß durch die Perforationen verbundene Teilstücke (2) mit einer Größe von maximal 5 cm² gebildet werden.

Weiterhin ist es bevorzugt, die Trocknung in Schritt d) mittels Gefriertrocknung durchzuführen, so dass eine besonders bevorzugte Ausführungsform gefriergetrocknete schichtförmige Biomatrices (1) betrifft.

Desweiteren ist es möglich, die in Schritt e) oder f) erhältlichen schichtförmigen Biomatrices vor dem Anbringen der Perforationen in die gewünschte geometrische Form oder Ausgestaltung zuzuschneiden, und gegebenenfalls mit einer farbigen Bedruckung zu versehen. Es ist beispielsweise denkbar, durch Bedrucken der schichtförmigen Biomatrix eine Art Schnitt- oder Trennmuster in Form einer farblichen Markierung aufzubringen, die einen geeigneten Verlauf für eine Auftrennung der Perforationen zur Abtrennung geeigneter Teilsegmente vorgibt.

Insbesondere in der therapeutischen Anwendung ist die Sterilisation der erfindungsgemäßen schichtförmigen Biomatrices von Bedeutung. Diese kann nach bekannten und gebräuchlichen Methoden durchgeführt werden.

Für die erfindungsgemäße Anwendung der schichtförmigen Biomatrix sind aus den vorstehend genannten Gründen einerseits gute Benetzungs- oder Adsorptionseigenschaften der verwendeten Trägermaterialien von Bedeutung.

Darüber hinaus ist aus den vorstehend genannten Gründen insbesondere eine hohe mechanische Stabilität, wie insbesondere eine hohe Reißfestigkeit der verwendeten Trägermaterialien maßgeblich. Die Nass-Reißfestigkeit der erfindungsgemäß bevorzugten Trägermaterialien, bestimmt nach DIN EN ISO 3376 vor dem Anbringen der erfindungsgemäßen Perforationen auf den schichtförmigen Trägermaterialien, beträgt bevorzugt mindestens 50 mN/mm Schichtdicke, bevorzugter 100 mN/mm, noch bevorzugter 200 mN/mm.

Bei Verwendung einer Methode zur Bestimmung der Reißfestigkeit mittels Stempel (interne Messmethode UV8801) werden an erfindungsgemäß perforierten und nicht perforierten Biomatrices mit Hilfe eines mechanischen Prüfgerätes (Zwick Materialprüfgerät B Z 2.5 / TN 1 S) ein Metallstempel mit kugelförmigem Kopf (25 mm im Durchmesser) auf das Vlies gedrückt und Weg und Kraft, die der Stempel zurücklegt bzw. ausübt, aufgezeichnet.

Für den Versuch wird die schichtförmige Biomatrix auf eine Größe von 8 x 8 cm zugeschnitten und in die Probenaufnahme des Gerätes eingebracht. Anschließend wird die Messung gestartet und der kugelförmige Stempel drückt auf die Probe, bis es zu einem Reißen des Materials kommt. Für die Bestimmung der Naßreißfestigkeit wird die Probe vor dem Start der Messung vollständig befeuchtet. Bei der Bestimmung der Trockenreißfestigkeit wird die Probe nicht befeuchtet, sondern trocken vermessen.

Die Kraft, bei dem es zu einem Riß des Materials kommt, wird mittels elektronischer Datenaufzeichnung aufgezeichnet, berechnet und ausgegeben. Bevorzugte Nass-Reißfestigkeiten der erfindungsgemäß bevorzugten Trägermaterialien vor dem Anbringen der erfindungsgemäßen Perforationen, bestimmt nach dieser internen Methode (UV 8801), betragen > 20 cN/mm Schichtdicke, bevorzugter > 30 cN/mm, noch bevorzugter > 40 cN/mm.

Durch die erfindungsgemäß angebrachten Perforationen wird die Reißfestigkeit erwartungsgemäß deutlich verringert, wodurch die vorstehend beschriebene Möglichkeit der einfachen Abtrennung von Teilsegmenten ermöglicht wird. So weist beispielsweise eine unperforierte gefriergetrocknete schichtförmige Biomatrix aus Collagen eine ca. vierfach so hohe Trocken-Reißfestigkeit, gemessen nach vorstehender Methode UV 8801, auf als eine entsprechende Collagen-Biomatrix, die mit den erfindungsgemäßen Perforationen unter Bildung quadratischer Teilstücke von 1 x 1 cm versehen wurde. Gegenüber perforierten Biomatrices mit Teilstücken von 0,5 x 0,5 cm Größe ist die Trocken-Reißfestigkeit, gemäß vorstehender Methode UV 8801, ca. um den Faktor 8 höher. Bei Alginat-basierten gefriergetrockneten Biomatrices kann durch eine erfindungsgemäße Perforation unter Bildung von 1 x 1 cm großen quadratischen Teilstücken entsprechend eine Reduzierung der Trocken-Reißfestigkeit ca. um den Faktor 12 beobachtet werden.

Desweiteren sind insbesondere auch aus ästhetischen Gründen, besonders in der kosmetischen, aber auch in der therapeutischen Anwendung solche Biomatrices erwünscht, deren Trägermaterial über eine hohe optische Dichte verfügt. Dabei bezeichnet optische Dichte die quantitative Einheit optische Dichte, gemessen als dekadischer Logarithmus des Quotienten von durchgelassener Lichtintensität zu eingestrahlter Lichtintensität, ermittelt mit einem Heiland SW Densitometer TD 03 an schichtförmigen Trägermaterialien mit einer Schichtdicke von 1 mm, gemessen vor dem Anbringen der erfindungsgemäßen Perforationen auf den schichtförmigen Trägermaterialien. Die Trägermaterialien der vorliegenden Erfindung weisen bevorzugt eine optische Dichte von ≥ 0,02, bevorzugter ≥ 0,03, noch bevorzugter ≥ 0,05 pro mm Schichtdicke auf.

Eine hohe optische Dichte ist dabei beispielsweise vorteilhaft für solche schichtförmigen Biomatrices, die mit farbigen Bedruckungen, z.B. in Form von ästhetischen Form- und Farbgestaltungen, Schriftzügen, Logos oder Anwendungserläuterungen bzw. den vorstehend erwähnten Markierungen versehen werden sollen.

Die erfindungsgemäßen schichtförmigen Biomatrices können einzeln abgepackt sein, was insbesondere in der therapeutischen bzw. pharmazeutischen aber auch in der professionellen kosmetischen Anwendung bevorzugt ist. Zuschnitte für die kosmetische Anwendung können auch in einer Mehrzahl neben- oder übereinander in Kontakt in einem geeigneten Behältnis oder einer geeigneten Verpackung vorliegen.

Die erfindungsgemäßen schichtförmigen Biomatrices dienen der äußeren kosmetischen sowie der äußeren und transdermalen pharmazeutischen Anwendung. Die äußere Anwendung erfolgt dabei in der Regel so, dass die schichtförmigen Biomatrices oder die daraus entlang der Perforationen vor der Anwendung abgetrennten Teilsegmente auf die zu behandelnden Körperpartien oder in der Wunde trocken aufgebracht und dort mit Wasser bzw. einer wässrigen Lösung, die einen oder mehrere Wirkstoffe und/oder einen oder mehrere Hilfsstoffe enthält (eine sogenannte Aktivatorlösung), angefeuchtet und rehydratisiert wird. Es ist jedoch auch möglich, die erfindungsgemäßen schichtförmigen Biomatrices oder Teilsegmente davon vor dem Aufbringen auf die zu behandelnde Körperpartie anzufeuchten oder bereits vorbefeuchtete schichtförmige Biomatrices in einer geeigneten Verpackung zur Verfügung zu stellen

Weiterer Gegenstand der vorliegenden Erfindung ist ein Pflege- oder Behandlungsset, enthaltend mindestens eine wie zuvor beschriebenen schichtförmigen Biomatrices.

Ferner betrifft die vorliegende Erfindung die Verwendung der schichtförmigen Biomatrices (1) zur kosmetischen und/oder therapeutischen Behandlung. Insbesondere ist Gegenstand der vorliegenden Erfindung, die vorstehend beschriebenen schichtförmigen Biomatrices als Mittel zur Behandlung von akuten Wunden wie z.B. traumatischen oder chirurgischen Wunden, z.B. Tumorwunden, sowie zur Behandlung von chronischen Wunden wie z.B. Dekubitus, Ulcus cruris, diabetischem Fußsyndrom etc. zu verwenden. Dabei können die schichtförmigen Biomatrices entweder als temporäre Auflage oder als Implantat verwendet werden. Erfindungsgemäß besonders bevorzugt ist dabei die Verwendung in der Behandlung von chronischen Wunden sowie die Verwendung als Hämostyptikum. Die erfindungsgemäßen schichtförmigen Biomatrices können dabei pharmazeutische Produkte oder Medizinprodukte darstellen.

Desweiteren können die vorstehend beschriebenen erfindungsgemäßen schichtförmigen Biomatrices auch in einer Vakuum-unterstützten Wundbehandlungstherapie, wie sie aus dem Stand der Technik bekannt ist und wie beispielsweise in der US 2007/0027414 beschrieben, verwendet werden. Dabei können die erfindungsgemäßen schichtförmigen Biomatrices in einer solchen Vakuum-Behandlung aufgrund der vorstehend beschriebenen hohen Flexibilität optimal in das Wundbett eingebracht werden und dort aufgrund ihrer guten Absorptions- und Hydratationseigenschaften den Abtransport des überschüssigen Wundsekrets positiv unterstützen. Dabei wird der Sekret-Transport durch das permeable Biomatrix-Material einerseits bereits durch die Wahl eines hydrophilen Matrixmaterials erreicht. Darüber hinaus verfügen insbesondere die erfindungsgemäß bevorzugten gefriergetrockneten Biomatrices aufgrund des Gefriertrocknungsprozesses über eine hohe Porosität, was den Flüssigkeitsdurchtritt zusätzlich erleichtert. Außerdem wird durch die erfindungsgemäßen Perforationen die Permeationskapazität der Biomatrices zusätzlich erhöht. Die Verwendung gefriergetrockneter poröser Materialien, ggf. auch solcher, die mit lochartigen Perforationen versehen sind, ist in der Vakuum-Therapie prinzipiell bereits bekannt. Dabei können jedoch insbesondere die erfindungsgemäßen perforierten schichtförmigen Biomatrices durch die Kombination der durch die spezielle Perforation erzielten hohen Flexibilität und damit räumlichen Modulierbarkeit in Kombination mit dem durch die Perforationen erzielten verbesserten Permeationseffekt besonders wirkungsvoll in einer Vakuum-unterstützten Wundbehandlungstherapie eingesetzt werden. Insbesondere schichtförmige Biomatrices aus Trägermaterialien, die an sich bereits einen positiven Einfluss auf den Wundheilungsverlauf aufweisen, wie beispielsweise die erfindungsgemäß bevorzugten Collagen-Trägermaterialien, sind dabei zur Verwendung in einer Vakuum-Therapie besonders geeignet und bevorzugt. Auch hier ist prinzipiell eine temporäre Verwendung möglich, worunter die schichtförmige Biomatrix wieder aus der Wunde entfernt oder im Verlauf der Behandlung partiell abgebaut und zusammen mit dem Wundsekret aus der Wunde ausgespült wird, oder die schichtförmige Biomatrix kann als Implantat in der Wunde verbleiben und wird dort entweder abgebaut bzw. verstoffwechselt oder im Verlauf der Wundepithelisierung mit Zellen durchwachsen und in den Körper eingebaut.

Die vorliegende Erfindung betrifft des weiteren auch eine Kombination, enthaltend mindestens eine der erfindungsgemäßen schichtförmigen Biomatrices sowie mindestens eine wässrige Lösung, die einen oder mehrere Wirkstoffe und/oder mindestens einen oder mehrere Hilfsstoffe enthält (eine sogenannte Aktivatorlösung), in einer zusammengehörenden, räumlichen Anordnung (Anwendungspaket, Set, Kit-of-Parts etc.). Dabei kann es sich bei der Wirkstofflösung beispielsweise um Lösungen von leicht-flüchtigen Wirk- und/oder Hilfsstoffen handeln, die aufgrund des Herstellverfahrens z.B. durch die Gefriertrocknung nicht in eine gefriergetrocknete Biomatrix eingebracht werden sollen bzw. können, wie z.B. gewisse Anteile ätherischer Öle, Parfums etc. Auch können solche Wirk-und/oder Hilfsstoffe enthalten sein, die eine befeuchtende Wirkung erzielen, die insbesondere bei der äußerlichen Anwendung auf der Haut erwünscht und bevorzugt ist, und die aufgrund dieser befeuchtenden Wirkung oder aufgrund von Hygroskopie-Neigungen nicht oder nur in geringen Mengen in die erfindungsgemäß bevorzugten gefriergetrockneten Biomatrices eingearbeitet werden können, da dadurch die Stabilität eventuell enthaltener feuchtigkeitslabiler Wirkstoffe nicht mehr aufrechterhalten werden kann. Grundsätzlich können in den Aktivatorlösungen einer oder mehrerer der vorstehend genannten Wirk-und/oder Hilfsstoffe enthalten sein.

Insbesondere können auch solche Wirkstofflösungen in den Kit-of-parts Ausgestaltungen enthalten sein, die zur therapeutischen Anwendung geeignet sind, wie z.B. zur Rehydrierung und Kühlung von Brandwunden, insbesondere bei der Akut- und Erstversorgung von Brandverletzungen oder zur Anwendung in einer feuchten Wundversorgung. Grundsätzlich sind solche Rehydrier- oder Wundbehandlungslösungen aus dem Stand der Technik bekannt. Es handelt sich dabei in der Regel um physiologische Lösungen oder Elektrolyt-haltige Lösungen, die ggf. weitere geeignete Wirkstoffe, wie oben ausgeführt, enthalten können. In den erfindungsgemäß bevorzugten Ausführungsformen handelt es sich in der Regel um wässrige Aktivator- oder Rehydratisierungslösungen, weswegen dann die Kombination in einer Kit-of-parts Anordnung mit einer erfindungsgemäßen schichtförmigen Biomatrix aus einem hydrophilen, offenporigen, saugfähigen Trägermaterial besonders bevorzugt ist. Eine offenporige schwammartige Schaumstruktur, wie sie insbesondere bei gefriergetrockneten Trägermaterialien vorhanden ist, ist besonders bevorzugt, da dadurch das Trägermaterial der schichtförmigen Biomatrix eine hohe Saugfähigkeit sowie bei Auswahl geeigneter Polymere, beispielsweise aus der Gruppe umfassend Collagen, Alginate und/oder Hyaluronsäure, idealerweise auch eine hohe Flüssigkeitsaufnahme- und -haltekapazität aufweist. Dies ist insbesondere bei Verwendung solcher Ausführungsformen zur Erstversorgung von Brandverletzten oder in der feuchten Wundbehandlung von besonderer Bedeutung, da es hierbei besonders wichtig ist, eine hohe Menge der Wirkstofflösung der Wunde zuzuführen und dort zu halten. Dies wird dadurch ermöglicht, dass die Flüssigkeit in die Poren des bevorzugten offenporigen, saugfähigen Polymerträgers eindringt und darin gehalten wird.

Ein weiterer wichtigen Effekt solcher bevorzugter Anwendungen liegt in der Möglichkeit der einfachen und großflächigen Abführung von Wärme, indem über die Verdunstung der Flüssigkeit eine kühlende Wirkung erzeugt wird. Eine Kühlung ist nicht nur vorteilhaft bei der Behandlung von Brandverletzungen oder Wunden, die aufgrund einer Entzündungsreaktion eine erhöhte Wärmeentwicklung aufweist, sondern auch bei geschädigter Haut z. B. in Folge von Sonnenbränden oder Sportverletzungen, sowie generell zur Linderung von schmerzhaften und unangenehmen Hautentzündungen, sowie zur Linderung von Nebenwirkungen von reizenden Kosmetikbehandlungen, wie z. B. Peeling-, Laser-, Fraxel- oder Resurfacingbehandlungen. Dabei ist insbesondere erwünscht, den Kühlungseffekt über einen längeren Zeitraum anhalten zu lassen, was insbesondere durch die oben beschriebenen, besonders bevorzugten hydrophilen, offenporigen, saugfähigen Trägermaterialien erreicht werden kann.

Ein hydrophobes Trägermaterial würde eine wässrige Wirkstofflösung nicht aufnehmen können. Die Verwendung eines hydrophoben Trägermaterials in Kombination mit einer hydrophoben Wirkstofflösung z. B. auf Basis von Fetten und Ölen ist aufgrund des Abschlusses der Wunde (der sogenannten Okklusion) mit Verhinderung der Transmission und Wärmeableitung nachteilig und daher unerwünscht.

Die Ausgestaltung solcher Kit-of-parts Kombinationen aus erfindungsgemäßer schichtförmiger Biomatrix einerseits und Wirkstofflösung andererseits kann dabei vorsehen, dass die beiden Bestandteile separat aus der Kit-of-parts Anordnung entnommen werden und außerhalb davon für die weitere Verwendung zusammengeführt werden. Es ist jedoch auch denkbar, dass eine Zusammenführung der Komponenten innerhalb der Kit-of-parts-Verpackung, z.B. in dafür vorgesehenen Kammern, selbst erfolgt und die hydratisierte Zusammensetzung sodann aus dieser direkt der weiteren kosmetischen oder pharmazeutischen äußerlichen oder transdermalen Verwendung zugeführt wird. Dies kann beispielsweise auch direkt durch den Endverbraucher durchgeführt werden.

## Patentansprüche

1. Schichtförmige Biomatrix (1) aus einem Trägermaterial, umfassend mindestens ein Polymer aus der Gruppe der strukturbildenden hydrophilen natürlichen Polymere und/oder der bioabsorbierbaren synthetischen und/oder modifizierten natürlichen Polymere, die aus regelmäßig geometrisch geformten, gleichmäßig angeordneten Teilstücken (2), die durch kontinuierliche, über die Fläche der schichtförmigen Biomatrix verlaufende Perforationen (3) abtrennbar miteinander verbunden sind, gebildet wird.

2. Schichtförmige Biomatrix nach Anspruch 1, worin die regelmäßig geformten, gleichmäßig angeordneten Teilstücke (2) im wesentlichen dreieck-, viereck-, waben-, kreis-, ellipsen-, herz- oder sternförmig sind.

3. Schichtförmige Biomatrix nach Anspruch 1, worin die Teilstücke (2) aus kontinuierlichen linearen, zueinander parallel angeordneten durchgehenden Perforationen (3a) und, die Perforationen (3a) schneidenden, kontinuierlichen linearen, zueinander parallel angeordneten durchgehenden Perforationen (3b) gebildet werden und worin die Abstände zwischen den parallel angeordneten Perforationen (3a) und/oder zwischen den, die Perforationen (3a) schneidenden, parallel angeordneten Perforationen (3b) jeweils gleich oder verschieden sind.

4. Schichtförmige Biomatrix nach einem der vorhergehenden Ansprüche, worin die regelmäßig geometrisch geformten Teilstücke (2) eine Größe von maximal 5 cm² aufweisen und worin die Größe der Teilstücke (2) jeweils gleich oder verschieden ist.

5. Schichtförmige Biomatrix nach einem der vorhergehenden Ansprüche, die neben dem Trägermaterial außerdem einen oder mehrere Wirk- und/oder Hilfsstoffe umfasst.

6. Schichtförmige Biomatrix nach einem der vorhergehenden Ansprüche, worin das Trägermaterial ein strukturbildendes hydrophiles natürliches Polymer aus der Gruppe der Collagene und/oder aus der Gruppe der Polysaccharide umfasst.

7. Schichtförmige Biomatrix nach Anspruch 5 oder 6, umfassend mindestens einen Wirkstoff der ausgewählt ist aus der Gruppe der blutstillenden Mittel und/oder aus der Gruppe der Wundbehandlungsmittel.

8. Schichtförmige Biomatrix nach einem der vorhergehenden Ansprüche, die gefriergetrocknet ist.

9. Verfahren zur Herstellung einer schichtförmigen Biomatrix gemäß einem der vorhergehenden Ansprüche, umfassend die Schritte
a) Herstellen einer wässrigen Suspension oder einer Lösung mindestens eines strukturbildenden Polymers;
b) gegebenenfalls Einmischen eines oder mehrerer Wirk- und/oder Hilfsstoffe;
c) Giessen der Mischung in eine geeignete Form;
d) Trocknen der Mischung;
e) Schneiden des aus Schritt d) erhältlichen getrockneten Formkörpers in Schichten von maximal 8 mm Dicke; gegebenenfalls
f) Schneiden der Schichten aus Schritt e) in die gewünschte geometrische Ausgestaltung der schichtförmigen Biomatrix (1);
g) Anbringen der kontinuierlichen, über die Fläche der schichtförmigen Biomatrix verlaufenden Perforationen (3) und dadurch Ausbildung von regelmäßig geometrisch geformten, gleichmäßig angeordneten Teilstücken (2) sowie gegebenenfalls
h) Sterilisieren und/oder Konfektionieren.

10. Verfahren nach Anspruch 9, worin in Schritt g) Perforationen (3) in Form kontinuierlicher linearer, zueinander parallel angeordneter durchgehender Perforationen (3a) und, die Perforationen (3a) schneidenden, kontinuierlicher linearer, zueinander parallel angeordneter durchgehender Perforationen (3b) jeweils in einem Schritt durch eine entsprechend geeignete Anzahl im gewünschten Abstand parallel angeordneter Perforationswerkzeuge angebracht werden, wobei der Abstand der parallel angeordneter Perforationswerkzeuge gleich oder verschieden ist.

11. Verfahren nach Anspruch 9 oder 10, worin in Schritt g) die Perforationen (3) so ausgebildet werden, daß durch die Perforationen verbundene Teilstücke (2) mit einer Größe von maximal 5 cm² gebildet werden.

12. Schichtförmige Biomatrix nach einem der Ansprüche 1 bis 8 zur Verwendung als pharmazeutisches Mittel.

13. Schichtförmige Biomatrix nach einem der Ansprüche 1 bis 8 zur Verwendung als Mittel zur Blutstillung und/oder als Mittel zur Behandlung von akuten und/oder chronischen Wunden und/oder als Implantat.

14. Schichtförmige Biomatrix nach Anspruch 13 zur Verwendung in einer Vakuumunterstützten Wundbehandlungstherapie.

15. Verwendung der schichtförmigen Biomatrix nach einem der Ansprüche 1 bis 8 als kosmetisches Mittel.

16. Kit-of-parts Kombination enthaltend mindestens eine schichtförmige Biomatrix nach einem der Ansprüche 1 bis 8 oder 12 bis 14 sowie mindestens eine wässrige Lösung, die einen oder mehrere Wirkstoffe und/oder einen oder mehrere Hilfsstoffe enthält.

## Claims

1. A stratiform biomatrix (1) of a carrier material comprising at least one polymer selected from the group of structure-forming hydrophilic natural polymers and/or bloabsorbable synthetic and/or modified natural polymers, that is formed from regularly geometrically shaped, uniformly arranged portions (2), which are separably connected to one another by continuous perforations (3) that run over the surface of the stratiform biomatrix.

2. A stratiform biomatrix according to claim 1, wherein the regularly shaped uniformly arranged portions (2) are substantially triangular, rectangular, honeycombed, circular, elliptical, heart-shaped or star-shaped.

3. The stratiform biomatrix according to claim 1, wherein the portions (2) are formed from continuous linear, straight-through perforations (3a), which are arranged parallel to one another, and from continuous linear, straight-through perforations (3b), which are arranged parallel to one another, and which are Intersecting each other and wherein the spacings between the perforations (3a), which arranged In parallel, and/or between the perforations (3b), which are arranged in parallel, and which are intersecting each other, are in each instance the same or different.

4. The stratiform biomatrlx according to any of the preceding claims, wherein the regularly geometrically shaped portions (2) have a size of at most 5 cm² and wherein the size of the portions (2) Is in each instance the same or different.

5. The stratiform biomatrix according to any of the preceding claims, comprising one or more active substances and/or auxiliary substances besides the carrier material.

6. The stratiform blomatrix according to any of the preceding claims, wherein the carrier material comprises a structure-forming hydrophilic natural polymer from the group of collagens and/or from the group of polysaccharides.

7. The stratiform blomatrix according to claim 5 or 6, comprising at least one active substance which Is selected from the group of hemostatic agents and/or from the group of wound-treatment agents.

8. The stratiform biomatrix according to any of the preceding claims, which is freeze-dried.

9. A process for the preparation of a stratiform biomatrix according to any of the preceding claims, comprising the steps:
a) producing an aqueous suspension or a solution of at least one structure-forming polymer;
b) optionally, mixing in one or more active substances and/or auxiliary substances;
c) pouring the mixture Into a suitable mould;
d) drying the mixture;
e) cutting the dried moulding obtainable from step d) Into layers of at most 8 mm thickness; optionally
f) cutting the layers from step e) into the desired geometrical configuration of the stratiform biomatrix (1);
g) putting the continuous perforations (3) that run over the surface of the stratiform biomatrix Into place and thereby forming regularly geometrically shaped, uniformly arranged portions (2); and also optionally
h) sterilizing and/or fabricating.

10. The process according to claim 9, wherein in step g) perforations (3) in the form of continuous linear straight-through perforations (3a), which are arranged parallel to one another, and continuous linear straight-through perforations (3b), which are arranged parallel to one another, and which are intersecting each other are put Into place in each instance in one step by means of an appropriately suitable number of perforating tools arranged in parallel with the desired spacing, the spacing between the perforating tools arranged in parallel being the same or different.

11. The process according to claim 9 or 10, wherein in step g) the perforations (3) are formed in such a way that portions (2) which are connected by the perforations are formed having a size of at most 5 cm².

12. The stratiform biomatrix according to any of the claims 1 to 8 for use as a pharmaceutical agent.

13. The stratiform biomatrix according to any of the claims 1 to 8 for use as a hemostatic agent and/or as an agent for treating acute and/or chronic wounds and/or as an implant.

14. The stratiform blomatrix according to claim 13 for use in a vacuum-assisted wound-treatment therapy.

15. Use of the stratiform biomatrix according to any of the claims 1 to 8 as a cosmetic agent.

16. A kit-of-parts combination containing at least one stratiform biomatrix according to any of the claims 1 to 8 or 12 to 14 and also at least one aqueous solution, that contains one or more active substances and/or one or more auxiliary substances.

## Revendications

1. Biomatrice en forme de couche (1) constituée d'un matériau de support comprenant au moins un polymère du groupe des polymères hydrophiles naturels formant des structures et/ou des polymères bloabsorbables synthétiques et/ou modifiés naturels, qui est formée par des parties (2) formées régulièrement géométriquement, arrangées uniformément, qui sont liés séparablement l'une à l'autre par des perforations (3) continues, qui s'étendent sur la superficie de la biomatrice en frome de couche.

2. Biomatrice en forme de couche selon la revendication 1, dans laquelle les parties (2) formées régulièrement, arrangées uniformément ont essentiellement une forme triangulaire, quadrangulaire, en nid d'abeilles, circulaire, elliptique, de coeur ou en étoile.

3. Biomatrice en forme de couche selon la revendication 1, dans laquelle les parties (2) sont formées par des perforations (3a) continues linéaires continuelles, arrangées parallèlement l'une à l'autre et des perforations (3b) continues linéaires continues, arrangées parallèlement l'une à l'autre coupant les perforations (3a) et dans laquelle les distances entre les perforations (3a) arrangées parallèlement et / ou entre des perforations (3b) arrangés parallèlement coupant les perforations (3a) sont respectivement Identiques ou différentes.

4. Biomatrice en forme de couche selon l'une des revendications précédentes, dans laquelle les parties (2) formées régulièrement géométriquement ont une taille de 5 cm² au maximum et dans laquelle la taille des parties (2) est respectivement identique ou différente.

5. Biomatrice en forme de couche selon l'une des revendications précédentes comprenant en outre un ou plusieurs substances actives et/ou des adjuvants en plus du matériau de support.

6. Biomatrice en forme de couche selon l'une des revendications précédentes, dans laquelle le matériau de support comprend un polymère hydrophile naturel formant des structures du groupe des collagènes et/ou du groupe des polysaccharides.

7. Biomatrice en forme de couche selon l'une des revendications 5 ou 6, comprenant au moins une substance active qui est sélectionnée parmi le groupe des agents hémostatiques et/ou du groupe des agents pour le traitement des blessures.

8. Blomatrice en forme de couche selon l'une des revendications précédentes, qui est lyophilisée,

9. Procédé de production d'une biomatrice en forme de couche selon l'une des revendications précédentes, comprenant les étapes
a) de la préparation d'une suspension aqueuse ou d'une solution d'au moins un polymère formant des structures ;
b) optionnellement le mélange d'un ou plusieurs substances actives et/ou des adjuvants ;
c) le moulage du mélange dans un moule approprié ;
d) le séchage du mélange ;
e) le découpage de l'article moulé obtenu de l'étape d) en couches qui ont une épaisseur qui est 8 mm au maximum ; optionnellement
f) le découplage des couches obtenues de l'étape e) en forme géométrique désirée de la biomatrice en forme de couche (1) ;
g) l'application des perforations (3) continues, qui s'étendent sur la superficie de la biomatrice en frome de couche et ainsi former des parties (2) formées régulièrement géométriquement, arrangées uniformément et optionnellement
h) la stérilisation et/ou la confection.

10. Procédé selon la revendication 9, dans lequel dans l'étape g) des perforations (3) sont appliquées en frome des perforations (3a) continues linéaires continuelles, arrangées parallèlement l'une à l'autre et des perforations (3b) continues linéaires continuelles, arrangées parallèlement l'une à l'autre coupant les perforations (3a) respectivement dans une étape par des outils de perforation disposés parallèlement en une distance désirée d'un nombre approprié, la distance des outils de perforation disposés parallèlement étant identique ou différente.

11. Procédé selon l'une des revendications 9 ou 10, dans lequel dans l'étape g) les perforations (3) sont formées tellement que les parties (2) liées par des perforations sont formées avec une taille de 5 cm² au maximum.

12. Biomatrice en forme de couche selon l'une des revendications 1 à 8 destinée à être utilisée en tant que produit pharmaceutique.

13. Biomatrice en forme de couche selon l'une des revendications 1 à 8 destinée à être utilisée en tant qu'agent hémostatique et/ou agent pour le traitement des blessures aiguës et/ou chroniques et/ou en tant qu'un implant.

14. Biomatrice en forme de couche selon la revendication 13 destinée à être utilisée dans une thérapie de traitement des blessures à l'aide du vide.

15. Utilisation de la biomatrice en forme de couche selon l'une des revendications 1 à 8 en tant qu'agent cosmétique.

16. Combinaison de jeu de pièces comprenant au moine une biomatrice en forme de couche selon l'une des revendications 1 à 8 ou 12 à 14 et au moins une solution aqueuse qui comprend un ou plusieurs substances actives et/ou un ou plusieurs adjuvants.
